# EUROPÄISCHE PATENTSCHRIFT

(11) **EP 1 751 153 B1**
(45) Veröffentlichungstag und Bekanntmachung des Hinweises auf die Patenterteilung: **03.12.2008**
(21) Anmeldenummer: 05740054.1
(22) Anmeldetag: 30.03.2005
(51) Int. Cl.: C07D 471/04, A61K 31/437, A61P 9/00

(54) **HETARYLOXY-SUBSTITUIERTE PHENYLAMINOPYRIMIDINE ALS RHO-KINASEHEMMER**
HETARYLOXY-SUBSTITUTED PHENYLAMINO PYRIMIDINES AS RHO KINASE INHIBITORS
PHENYLAMINOPYRIMIDINES SUBSTITUEES PAR HETARYLOXY, UTILISEES COMME INHIBITEURS DE LA RHO-KINASE

(30) Priorität: 08.04.2004 DE 102004017438
(43) Veröffentlichungstag der Anmeldung: 14.02.2007
(73) Patentinhaber: Bayer HealthCare AG, 51368 Leverkusen (DE)
(72) Erfinder: SCHIROK, Hartmut, 42287 Wuppertal (DE); RADTKE, Martin, 46099 Erkrath (DE); MITTENDORF, Joachim, 42113 Wuppertal (DE); KAST, Raimund, 42349 Wuppertal (DE); STASCH, Johannes-Peter, 42651 Solingen (DE); GNOTH, Mark, Jean, 40822 Mettmann (DE); MÜNTER, Klaus, 42489 Wülfrath (DE); LANG, Dieter, 42553 Velbert (DE); FIGUEROA, PEREZ, Santiago, 51373 Leverkusen (DE); THUTEWOHL, Michael, 9470 Buchs (CH); BENNABI, Samir, 69300 Caluire et Cuire (FR); EHMKE, Heimo, 20251 Hamburg (DE)
(86) Internationale Anmeldenummer: PCT/EP2005/003294
(87) Internationale Veröffentlichungsnummer: WO 2005/097790

(56) Entgegenhaltungen:
- WO-A-03/062225
- WO-A-03/062227
- WO-A-03/106450
- WO-A-20/04039796

## Beschreibung

Die Erfindung betrifft hetaryloxy-substituierte Phenylaminopyrimidine, ein Verfahren zu ihrer Herstellung sowie ihre Verwendung zur Herstellung von Arzneimitteln zur Behandlung und/oder Prophylaxe von Krankheiten bei Menschen und Tieren, insbesondere von kardiovaskulären Erkrankungen.

Ein Anstieg der intrazellulären Calcium-Konzentration ist ein Hauptauslöser für die Kontraktion der Gefäßmuskulatur (Somlyo, A.P. und Himpens, B. FASEB J. 1989, 3, 2266-2276). Dies geschieht in erster Linie durch Agonisten wie z.B. Phenylephrin oder Thromboxan A2, die nach Stimulierung der Phosphatidylinositolkaskade die Freisetzung von Calcium aus dem sarkoplasmatischen Retikulum bewirken. Die Erhöhung des intrazellulären Calciums aktiviert die MLC-Kinase (Myosin-Leichte-Ketten-Kinase), die die MLC-Untereinheiten des Myosinmoleküls phosphoryliert (Kamm, K.H. und Stull, J.T., Annu. Rev. Pharmacol. Toxicol. 1985, 25, 593-603). MLC-Phosphorylierung induziert die Glattmuskelkontraktion, MLC-Dephosphorylierung nach einer Reduktion der intrazellulären Calciumkonzentration resultiert in der Relaxation des Gefäßes.

Neben der Calcium-abhängigen MLC-Phosphorylierung existiert noch ein weiterer zentraler aber Calcium-unabhängiger Regulationsmechanismus des Gefäßtonus. Hierbei handelt es sich um den Rho/Rho-Kinase-Signalweg (Noda, M. et al., FEBS Lett. 1995, 367, 246-250; Uehata, M. et al., Nature 1997, 389, 990-994; Fukata, Y. et al., Trends in Pharmacological Sciences 2001, 22, 32-39). Binden Agonisten wie z.B. Phenylephrin oder Thromboxan A2 an ihre Rezeptoren, so führt dies zur Aktivierung der kleinen G-Proteine Rho, die dann mit der Rho-Kinase interagieren und diese aktivierten. Die aktivierte Rho-Kinase inhibiert die Myosin-Phosphatase, nachdem sie eine Untereinheit des Enzyms phosphoryliert hat. Gleichzeitig phosphoryliert Rho-Kinase MLC an der Stelle, die auch von der MLC-Kinase phosphoryliert wird. Eine Hemmung der Myosin-Phosphatase sowie der Phosphorylierung von MLC induziert die Kontraktion der Gefäßmuskulatur. Im Gegensatz dazu führt eine Hemmung der Rho-Kinase zu einer Gefäßrelaxation. Inhibitoren der Rho-Kinase bewirken daher eine Senkung des Blutdruckes und eine Steigerung des koronaren Blutflusses.

Darüber hinaus führen Inhibitoren der Rho-Kinase zu einer Hemmung des Wachstums von Tumorzellen und Metastasen (Itoh et al. Nat. Med. 1999, 5, 221; Somlyo et al. Biochem. Biophys. Res. Commun. 2000, 269, 652) und inhibieren die Angiogenese (Uchida et al. Biochem. Biophys. Res. Commun. 2000,269, 633; Gingras et al. Biochem. J. 2000, 348 Bd. 2, 273).

Den erfindungsgemäßen Verbindungen ähnliche Strukturen sind aus anderen Indikationen bekannt. So offenbart beispielsweise US 2001/0020030 A1 substituierte Thienopyridine und Thienopyrimidine zur Behandlung von inflammatorischen Erkrankungen, WO 02/32872 offenbart stickstoffhaltige aromatische Ringverbindungen als Inhibitoren der Neovaskularisation.

Den erfindungsgemäßen Verbindungen ähnliche Strukturen sind weiterhin in WO 03/062225, WO 03/062227, WO 03/106450 und WO 04/039796 als Rho-Kinase-Inhibitoren zur Behandlung von Krebs und kardiovaskulären Erkrankungen beschrieben. Allerdings zeigte es sich, dass diese Verbindungen hinsichtlich ihrer in vivo-Eigenschaften wie beispielsweise ihrem Verhalten in der Leber, ihrem pharmakologischen Verhalten oder ihrem Metabolisierungsweg Nachteile aufweisen.

Es war daher die Aufgabe der vorliegenden Erfindung, weitere hetaryloxy-substituierte Phenylaminopyrimidine bereitzustellen, welche als Rho-Kinase-Inhibitoren wirken, aber nicht die vorstehend aufgeführten Nachteile aus dem Stand der Technik aufweisen.

Gegenstand der vorliegenden Erfindung sind Verbindungen der Formel 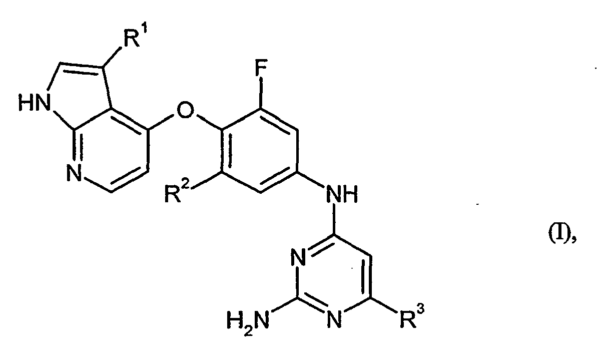 worin
- R¹: Chlor, Brom, Cyano, Methyl, Ethyl, Hydroxyethyl, Methoxyethyl oder Cyclopropyl bedeutet,
- R²: Wasserstoff oder Fluor bedeutet,
- R³: Wasserstoff, Chlor, Trifluormethyl oder Pentafluorethyl bedeutet,
und ihre Salze, Hydrate, Hydrate der Salze und Solvate.

Erfindungsgemäße Verbindungen sind die Verbindungen der Formel (I) und deren Salze, Solvate und Solvate der Salze; die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können teilweise in Abhängigkeit von ihrer Struktur in stereoisomeren Formen (Enantiomere, Diastereomere) existieren. Die Erfindung umfasst deshalb auch die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Efhansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Bevorzugt sind Verbindungen der Formel (I), worin
- R¹: Chlor, Cyano, Methyl, Ethyl, Hydroxyethyl, Methoxyethyl oder Cyclopropyl bedeutet,
- R²: Wasserstoff oder Fluor bedeutet,
- R³: Wasserstoff, Chlor, Trifluormethyl oder Pentafluorethyl bedeutet,
und ihre Salze, Hydrate, Hydrate der Salze und Solvate.

Bevorzugt sind auch Verbindungen der Formel (I), worin
- R¹: Chlor, Cyano, Methyl, Ethyl, Hydroxyethyl, Methoxyethyl oder Cyclopropyl bedeutet,
- R²: Wasserstoff oder Fluor bedeutet,
- R³: Wasserstoff, Chlor oder Trifluormethyl bedeutet,
und ihre Salze, Hydrate, Hydrate der Salze und Solvate.

Besonders bevorzugt sind Verbindungen der Formel (I), worin
- R¹: Cyano, Methyl oder Hydroxyethyl bedeutet,
- R²: Wasserstoff oder Fluor bedeutet,
- R³: Chlor oder Trifluormethyl bedeutet,
und ihre Salze, Hydrate, Hydrate der Salze und Solvate.

Weiterer Gegenstand der vorliegenden Erfindung ist ein Verfahren zur Herstellung der Verbindungen der Formel (I), das dadurch gekennzeichnet ist, dass man

Verbindungen der Formel (II) 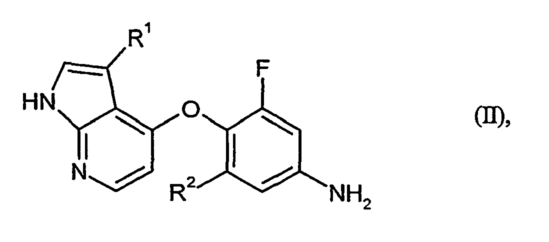 worin
R¹ und R² die oben angegebene Bedeutung aufweisen,
mit Verbindungen der Formel (III) 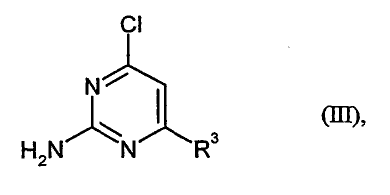 worin
- R³: die oben angegebene Bedeutung aufweist, umsetzt.

Die Umsetzung erfolgt im Allgemeinen in wässriger salzsaurer Lösung, bevorzugt in einem Temperaturbereich von 70°C bis 110°C bei Normaldruck.

Zur Herstellung von Verbindungen der Formel (II) werden beispielsweise in Verbindungen der Formel (IV) 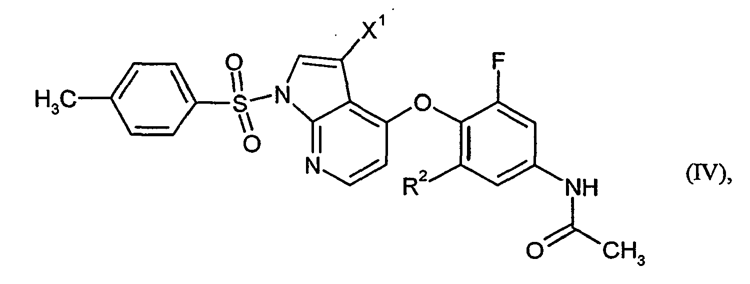 worin
- R²: die oben angegebene Bedeutung aufweist, und
- X¹: Halogen, bevorzugt Brom oder Chlor, bedeutet,
über metallorganische Reaktionen, wie z. B. Suzuki- oder Negishi-Reaktionen, oder andere dem Fachmann bekannte Palladium-katalysierte Reaktionen weitere Reste R¹ eingerührt. Die Synthesewege für die verschiedenen Reste R¹ sind ausführlich bei den Beispielen im experimentellen Teil beschrieben. Anschießend werden die Acyl- und Tosylat-Schutzgruppe nach üblichen, dem Fachmann bekannten Methoden abgespalten.

Zur Herstellung der Verbindungen der Formel (IV) werden beispielsweise Verbindungen der Formel (V) 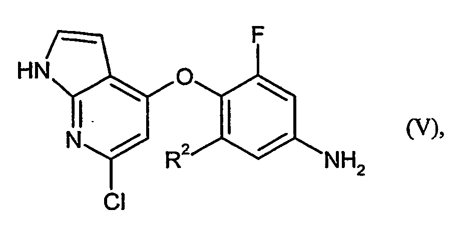 worin
- R²: die oben angegebene Bedeutung aufweist,
in einer ersten Stufe mit Palladium auf Aktivkohle in Ethanol bei 50°C unter 2 bar Wasserstoff-Druck hydriert, um den Chlor-Substituenten abzuspalten.

In einer zweiten Stufe wird eine Acetyl-Schutzgruppe am Anilin durch die Umsetzung mit Acetylchlorid in Dichlormethan in Gegenwart von Triethylamin in einem Temperaturbereich von 0°C bis Raumtemperatur bei Normaldruck eingeführt.

In einer dritten Stufe wird dann der Rest X¹ eingeführt:

Im Falle von X¹ = Brom erfolgt die Umsetzung in einem Eisessig/Dichlormethan-Gemisch durch Zugabe von Brom in einem Temperaturbereich von -10°C bis 10°C bei Normaldruck.

Im Falle von X¹ = Chlor erfolgt die Umsetzung mit N-Chlorsuccinimid in THF durch Zugabe von wässriger Salzsäure in einem Temperaturbereich von Raumtemperatur bis zum Rückfluss des Lösungsmittels bei Normaldruck.

In einer anschließenden vierten Stufe erfolgt die Einführung der Tosylat-Schutzgruppe am Pyrrol-Stickstoff des Pyrrolopyridins durch Deprotonierung mit Butyllithiumlösung in THF in einem Temperaturbereich von -78°C bis -50°C bei Normaldruck und anschließende Umsetzung mit Toluolsulfonsäurechlorid in THF unter Erwärmung auf Raumtemperatur.

Zur Herstellung der Verbindungen der Formel (V) werden beispielsweise Verbindungen der Formel (VI) 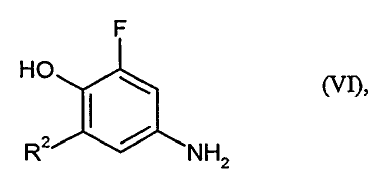 worin
- R²: die oben angegebene Bedeutung aufweist,
mit der Verbindung der Formel (VII) 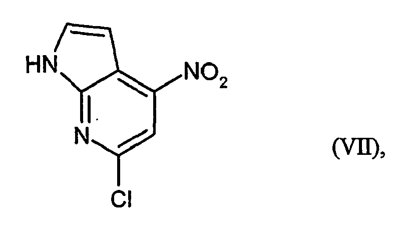 umgesetzt.

Die Umsetzung erfolgt in Substanz in Gegenwart von Kaliumhydroxid als Base in der Schmelze bei einer Temperatur von 200°C bis 280°C oder vorzugsweise in einem inerten Lösungsmittel wie beispielsweise *N,N*-Dimethylformamid, *N*-Methylpyrrolidon, Dimethylsulfoxid oder Nitrobenzol in Gegenwart einer Base wie beispielsweise Kaliumcarbonat, Kaliumhydroxid, Kalium-*tert*-butylat oder Natriumhydrid bei einer Temperatur von 120°C bis 280°C.

Die Verbindungen der Formel (III), (VI) und (VII) sind dem Fachmann an sich bekannt oder lassen sich nach üblichen literaturbekannten Verfahren herstellen.

Die Herstellung der erfindungsgemäßen Verbindungen kann durch folgendes Syntheseschema verdeutlicht werden.

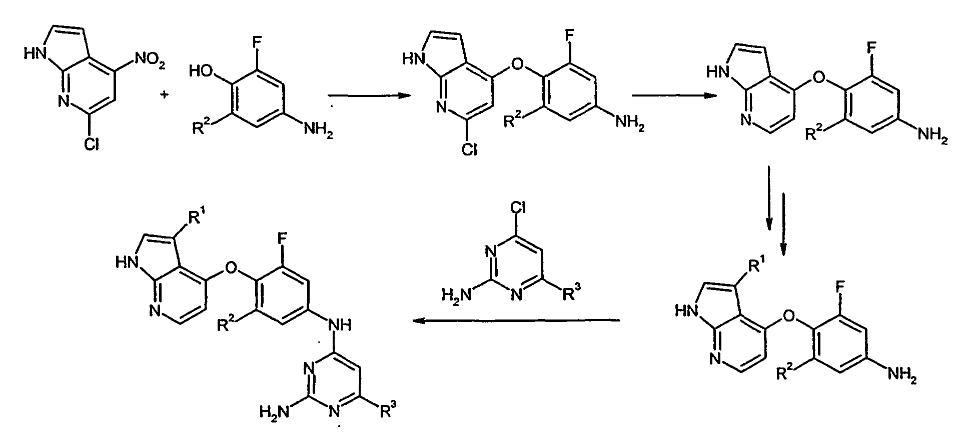

Die erfindungsgemäßen Verbindungen zeigen ein nicht vorhersehbares, wertvolles pharmakologisches und pharmakokinetisches Wirkspektrum.

Sie eignen sich daher zur Verwendung als Arzneimittel zur Behandlung und/oder Prophylaxe von Krankheiten bei Menschen und Tieren.

Die pharmazeutische Wirksamkeit der erfindungsgemäßen Verbindungen lässt sich durch ihre Wirkung als Rho-Kinase-Inhibitoren erklären.

Weiterer Gegenstand der vorliegenden Erfindung ist der Einsatz der erfindungsgemäßen Verbindungen zur Behandlung und/oder Prophylaxe von Erkrankungen, vorzugsweise von kardiovaskulären Erkrankungen.

Die erfindungsgemäßen Verbindungen sind geeignet für die Prophylaxe und/oder Behandlung von kardiovaskulären Erkrankungen wie beispielsweise Bluthochdruck und Herzinsuffizienz, stabiler und instabiler Angina pectoris, peripheren und kardialen Gefäßerkrankungen, von Arrhythmien, von thromboembolischen Erkrankungen und Ischämien wie Myokardinfarkt, Hirnschlag, transitorischen und ischämischen Attacken, peripheren Durchblutungsstörungen, Subarachnoidalblutungen, Verhinderung von Restenosen wie beispielsweise nach Thrombolysetherapien, percutanen transluminalen Angioplastien (PTA), percutanen transluminalen Koronarangioplastien (PTCA), Bypass sowie zur Prophylaxe und/oder Behandlung von Arteriosklerose, Alzheimer, Niereninsuffizienz, Glaukom, asthmatischen Erkrankungen, COPD und Krankheiten des Urogenitalsystems wie beispielsweise Prostatahypertrophie, erektiler Dysfunktion, weiblicher sexueller Dysfunktion, Osteoporose, Gastroparese und Inkontinenz.

Weiterhin können die erfindungsgemäßen Verbindungen zur Prophylaxe und/oder Behandlung von Krebserkrankungen, insbesondere von Tumoren eingesetzt werden.

Im Rahmen der vorliegenden Erfindung umfasst die Definition von Tumoren sowohl benigne, wie auch maligne Tumore und damit beispielsweise auch benigne Neoplasien, Dysplasien, Hyperplasien, wie auch Neoplasien mit Metastasenbildung. Weitere Beispiele für Tumore sind Karzinom, Sarkome, Karzinosarkome, Tumore der blutbildenden Organe, Tumore des Nervengewebes z.B. des Gehirns oder Tumore von Hautzellen. Bei der Tumorbildung kommt es zur unkontrollierten oder unzureichend kontrollierten Zellteilung. Der Tumor kann örtlich begrenzt sein, er kann aber auch das umliegende Gewebe infiltrieren und sich dann durch das lymphatische System oder durch den Blutstrom an einem neuen Ort festsetzen. Somit gibt es primäre und sekundäre Tumore. Primäre Tumore sind ursprünglich in dem Organ entstanden, in dem sie gefunden werden. Sekundäre Tumore haben sich durch Metastasenbildung in einem anderen Organ festgesetzt und sich dann an ihrem neuen Ort ausgebreitet.

Weiterer Gegenstand der vorliegenden Erfindung ist die Verwendung der erfindungsgemäßen Verbindungen zur Prophylaxe und/oder Behandlung von Erkrankungen, insbesondere der zuvor genannten Krankheitsbilder.

Weiterer Gegenstand der vorliegenden Erfindung ist die Verwendung der erfindungsgemäßen Verbindungen zur Herstellung eines Arzneimittels zur Prophylaxe und/oder Behandlung von Erkrankungen, insbesondere der zuvor genannten Krankheitsbilder.

Weiterer Gegenstand der vorliegenden Erfindung ist ein Verfahren zur Prophylaxe und/oder Behandlung von Erkrankungen, insbesondere der zuvor genannten Erkrankungen, unter Verwendung einer kardiovaskulär wirksamen Menge der erfindungsgemäßen Verbindung.

Weiterer Gegenstand der vorliegenden Erfindung sind Arzneimittel, enthaltend eine erfindungsgemäße Verbindung in Kombination mit einem oder mehreren weiteren Wirkstoffen, insbesondere zur Prophylaxe und/oder Behandlung der zuvor genannten Erkrankungen.

Die erfindungsgemäße Verbindung kann systemisch und/oder lokal wirken. Zu diesem Zweck kann sie auf geeignete Weise appliziert werden, wie z.B. oral, parenteral, pulmonal, nasal, sublingual, lingual, buccal, rectal, transdermal, conjunctival, otisch, als Stents oder als Implantat.

Für diese Applikationswege kann die erfindungsgemäße Verbindung in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nichtüberzogene oder überzogene Tabletten, beispielsweise mit magensaftresistenten, sich verzögert auflösenden oder unlöslichen Überzügen, die die Freisetzung der erfindungsgemäßen Verbindungen kontrollieren), in der Mundhöhle schnell zerfallende Tabletten oder Filme/Oblaten, Kapseln, Dragees, Granulate, Pellets, Pulver, Emulsionen, Suspensionen, Aerosole oder Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen/-lösungen, Sprays; lingual, sublingual oder buccal zu applizierende Tabletten oder Kapseln, Suppositorien, Ohren- und Augenpräparationen, Vaginalkapseln, wässrige suspensionen (Lotionen, Schüttelmixturen), lipophile Suspensionen, Salben, Cremes, Milch, Pasten, Streupuder, Stents oder Implantate.

Die erfindungsgemäßen Verbindungen können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und/oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich sowohl in der Human- als auch in der Veterinärmedizin als vorteilhaft erwiesen, die erfindungsgemäße Verbindung in Gesamtmengen von etwa 0.01 bis etwa 700, vorzugsweise 0.01 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält die erfindungsgemäße Verbindung vorzugsweise in Mengen von etwa 0.1 bis etwa 80, insbesondere 0.1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

| | |
|---|---|
| DC | Dünnschichtchromatographie |
| DCI | direkte chemische Ionisation (bei MS) |
| DCM | Dichlormethan |
| DIEA | *N,N*-Diisopropylethylamin |
| DMA | *N,N*-Dimethylacetamid |
| DMF | *N,N*-Dimethylformamid |
| DMSO | Dimethylsulfoxid |
| d. Th. | der Theorie |
| EE | Ethylacetat (Essigsäureethylester) |
| EI | Elektronenstoß-Ionisation (bei MS) |
| ESI | Elektrospray-Ionisation (bei MS) |
| Fp. | Schmelzpunkt |
| ges. | gesättigt |
| h | Stunde |
| HATU | *O*-(7-Azabenzotriazol-1-yl)-*N,N,N,N'*-tetramethyluronium-hexafluorphosphat |
| HOAT | 3H-[1,2,3]-Triazol[4,5-b]pyridin-3-ol |
| HOBt | 1-Hydroxy-1H-benzotriazol x H₂O |
| HPLC | Hochdruck-, Hochleistungsflüssigchromatographie |
| konz. | konzentriert |
| LC-MS | Flüssigchromatographie-gekoppelte Massenspektroskopie |
| LDA | Lithiumdiisopropylamid |
| min | Minuten |
| MPLC | Mitteldruck-, Mittelleistungsflüssigchromatographie |
| MS | Massenspektroskopie |
| NMR | Kernresonanzspektroskopie |
| org. | organisch |
| proz. | prozentig |
| RF | Rückfluss |
| R_{f} | Retentionsfaktor (bei DC) |
| RP-HPLC | Reverse Phase HPLC |
| RT | Raumtemperatur |
| Rₜ | Retentionszeit (bei HPLC) |
| TFA | Trifluoressigsäure |
| THF | Tetrahydrofuran |

### HPLC-, LCMS- und GCMS-Methoden:

**Methode 1 (LC/MS):** Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50% ige Ameisensäure, Eluent B: 11 Acetonitril + 0.5 ml 50% ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 2 (LC/MS):** Instrument: Micromass Quattro LCZ mit HPLC Agilent Serie 1100; Säule: Phenomenex Synergi 2µ Hydro-RP Mercury 20 mm x 4 mm; Eluent A: 11 Wasser + 0.5 ml 50% ige Ameisensäure, Eluent B: 1 1 Acetonitril + 0.5 ml 50% ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208- 400 nm.

**Methode 3 (LC/MS):** Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50% ige Ameisensäure, Eluent B: 11 Acetonitril + 0.5 ml 50% ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

**Methode 4 (HPLC):** Instrument: HP 1100 mit DAD-Detektion; Säule: Kromasil 100 RP-18, 60 mm x 2.1 mm, 3.5 µm; Eluent A: 5 ml Perchlorsäure (70%ig)/l Wasser, Eluent B: Acetonitril; Gradient: 0 min 2%B, 0.5 min 2%B, 4.5 min 90%B, 6.5 min 90%B, 6.7 min 2%B, 7.5 min 2%B; Fluss: 0.75 ml/min; Ofen: 30°C; UV-Detektion: 210 nm.

**Methode 5 (LC-MS):** Instrument: Micromass Platform LCZ mit HPLC Agilent Serie 1100; Säule: Thermo HyPURITY Aquastar 3µ 50 mm x 2.1 mm; Eluent A: 11 Wasser + 0.5 ml 50% ige Ameisensäure, Eluent B: 1 1 Acetonitril + 0.5 ml 50% ige Ameisensäure; Gradient: 0.0 mim 100%A → 0.2 min 100%A → 2.9 min 30%A → 3.1 min 10%A → 5.5 min 10%A; Ofen: 50°C; Fluss: 0.8 ml/min; UV-Detektion: 210 nm.

**Methode 6 (LC/MS):** Instrument: Micromass Platform LCZ mit HPLC Agilent Serie 1100; Säule: Phenomenex Synergi 2µ Hydro-RP Mercury 20 mm x 4 mm; Eluent A: 1 1 Wasser + 0.5 ml 50% ige Ameisensäure, Eluent B: 1 1 Acetonitril + 0.5 ml 50% ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Präparative HPLC:** Säule: YMC Gel ODS-AQ S-5 / 15 µM, 250 mm x 30 mm, Eluent A: Wasser, Eluent B: Acetonitril; Gradient: 0.00 min 30%B → 3.00 min 30%B → 34.0 min 95%B → 38.0 min 30%B; Temp.: Raumtemperatur; Fluss: 50 ml/min; UV-Detektion.

### Ausgangsverbindungen

### Beispiel 1A

### 1H-Pyrrolo[2,3-b]pyridin-7-oxid

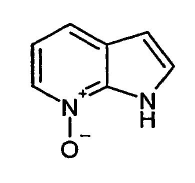

(Antonini, Ippolito; Claudi, Francesco; Cristalli, Gloria; Franchetti, Palmarisa; Grifantini, Mario; Martelli, Sante; J. Med. Chem. 1982, 25(10), 1258-1261) 540 g (2.35 mol) 3-Chlorperbenzoesäure werden in 6.11 Dichlormethan gelöst und abgeschiedenes Wasser wird abgetrennt. Die organische Phase wird über Natriumsulfat getrocknet und auf 0°C gekühlt. Dann gibt man eine Lösung aus 163 g (1.38 mol) 1H-Pyrrolo[2,3-b]pyridin (Hands, D.; Bishop, B.; Cameron, M.; Edwards, T.S.; Cottrell, I.F.; Wright, S.H.B.; Synthesis 1996 (7), 877-882) in 1.00 1 Dichlormethan zu und lässt die Temperatur auf Raumtemperatur ansteigen. Nach 2 Stunden gibt man soviel Methanol zu, dass sich der gebildete Niederschlag wieder auflöst. Die Mischung wird über Kieselgel filtriert (Eluens: Dichlormethan/Methanol 95 : 5). Die Produktfraktionen werden nach Einengen am Hochvakuum getrocknet.
Ausbeute; 145 g (75% d. Th.)
HPLC (Methode 4): Rₜ = 2.02 min.
MS (ESI pos.): m/z = 135 (M+H)⁺, 152 (M+NH₄)⁺, 269 (2M+H)⁺.
¹H-NMR (DMSO-d₆, 200 MHz): δ = 6.58 (d, 1H), 7.07 (dd, 1H), 7.48 (d, 1H), 7.65 (d, 1H), 8.17 (d, 1H), 12.42-12.64 (br. s, 1H).

### Beispiel 2A

### 4-Nitro-1H-pyrrolo[2,3-b]pyridin-7-oxid

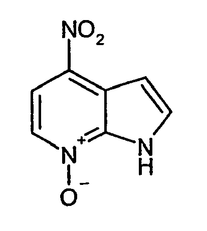

Eine Durchführung größerer Ansätze als die fünffache Menge der beschriebenen Größe ist auf grund der Ergebnisse der Differentialthermoanalyse nicht empfehlenswert.

Eine Lösung von 20.0 g (149 mmol) 1H-Pyrrolo[2,3-b]pyridin-7-oxid in 160 ml Trifluoressigsäure wird auf Raumtemperatur gekühlt. Anschließend tropft man langsam 69.3 ml 65%ige Salpetersäure zu und lässt 2 Stunden bei Raumtemperatur rühren. Man gießt auf Eis und stellt mit 45%iger Natriumhydroxidlösung einen pH-Wert von 8-9 ein. Der Niederschlag wird abgesaugt und mit Wasser gewaschen. Es werden die Rohprodukte aus 4 Ansätzen der beschriebenen Größe und einem analog durchgeführten 13 g-Ansatz vereinigt und gemeinsam gereinigt. Die Rohprodukte werden in Wasser aufgeschlämmt und mit 2N Natriumhydroxidlösung auf pH8-9 eingestellt. Nach 10 min Rühren wird der Niederschlag abgesaugt und im Hochvakuum getrocknet. (Schneller, Stewart W.; Luo, Jiann-Kuan; J. Org. Chem. 1980, 45, 4045-4048.)
Ausbeute: 29.7 g (24% d. Th.)
HPLC (Methode 4): Rₜ = 3.02 min.
MS (ESI pos.): m/z = 180 (M+H)⁺, 197 (M+NH₄)⁺, 359 (2M+H)⁺.
¹H-NMR (DMSO-d₆, 200 MHz): δ = 7.03 (d, 1H), 7.80 (d, 1H), 8.03 (d, 1H), 8.31 (d, 1H), 13.22-13.41 (br. s, 1H).

### Beispiel 3A

### 6-Chlor-4-nitro-1H-pyrrolo[2,3-b]pyridin

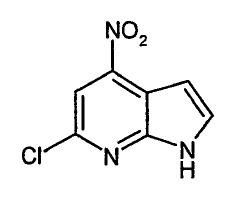

5.00 g (27.9 mmol) 4-Nitro-1H-pyrrolo[2,3-b]pyridin-7-oxid und 11.8 ml (55.8 mmol) Hexamethyldisilazan werden in 290 ml THF unter einer Argonatmosphäre vorgelegt. 10.8 ml (140 mmol) Chlorameisensäuremethylester werden bei RT zugegeben. Die Lösung wird über Nacht bei RT gerührt. Die Reaktionslösung wird über eine Kieselgelkartusche filtriert und mit Dichlormethan/Methanol 10:1 nachgewaschen.
Ausbeute: 2.8 g (70% d. Th.)
LC-MS (Methode 3): Rₜ = 2.15 min.
MS (ESI pos.): m/z = 198 (M+H)⁺.
¹H-NMR (DMSO-d₆, 200 MHz): δ = 7.00 (mc, 1H), 7.97 (s, 1H), 8.00 (t, 1H), 12.79 (s, 1H).

### Beispiel 4A

### 4-[(6-Chlor-1H-pyrrolo[2,3-b]pyridin-4-yl)oxy]-3,5-difluoranilin

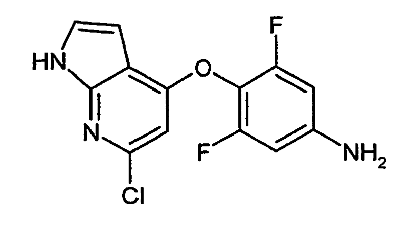

664 mg (3.36 mmol) 6-Chlor-4-nitro-1H-pyrrolo[2,3-b]pyridin, 1.39 g (10.1 mmol) pulverisiertes Kaliumcarbonat und 877 mg (5.04 mmol) Natriumdithionit werden in 10 ml DMSO suspendiert. Die Mischung wird entgast, und 915 mg (5.04 mmol) 4-Amino-2,6-difluorphenol-Hydrochlorid werden zugesetzt. Man erhitzt 4 Stunden auf 120°C. Nach Zugabe von Essigsäureethylester wird über Celite abgesaugt und mit Essigsäureethylester nachgewaschen. Das Filtrat wird dreimal mit gesättigter Natriumhydrogencarbonatlösung und mit gesättigter Natriumchloridlösung geschüttelt. Man trocknet über Natriumsulfat und entfernt das Lösungsmittel im Vakuum. Der Rückstand wird durch Säulenchromatographie gereinigt (Kieselgel 60, Eluens: Dichlormethan/Methanol = 50 : 1).
Ausbeute: 356 mg (36% d. Th.)
LC-MS (Methode 1): Rₜ = 2.05 min.
MS (ESI pos.): m/z = 296 (M+H)⁺.
¹H-NMR (DMSO-d₆, 400 MHz): δ = 5.84 (s, 2H), 6.28 (mc, 1H), 6.38-6.41 (m, 3H), 7.42 (mc, 1H), 12.02 (s, 1H).

### Beispiel 5A

### 3,5-Difluor-4-(1H-pyrrolo[2,3-b]pyridin-4-yloxy)anilin

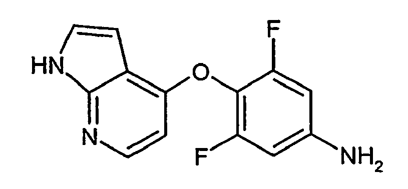

Analog 3-Fluor-4-(1H-pyrrolo[2,3-b]pyridin-4-yloxy)anilin wird die Titelverbindung durch katalytische Hydrierung von 408 mg (1.38 mmol) 4-[(6-Chlor-1H-pyrrolo[2,3-b]pyridin-4-yl)oxy]-3,5-difluoranilin gewonnen.
Ausbeute: 360 mg (100% d. Th.)
LC-MS (Methode 1): Rₜ = 1.46 min.
MS (ESI pos.): m/z = 262 (M+H)⁺.
¹H-NMR (DMSO-d₆ 400 MHz): δ = 5.77 (br. s, 1H), 6.28 (dd, 1H), 6.34-6.40 (m, 3H), 7.37 (dd, 1H), 8.06 (d, 1H), 11.76 (br. s, 1H).

### Beispiel 6A

### N-[3,5-Difluor-4-(1H-pyrrolo[2,3-b]pyridin-4-yloxy)phenyl]acetamid

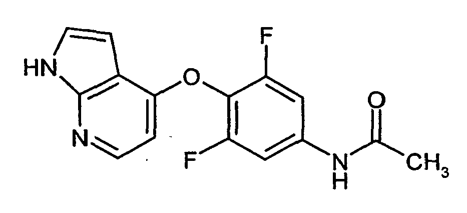

1.60 g (6.14 mmol) 3,5-Difluor-4-(1H-pyrrolo[2,3-b]pyridin-4-yloxy)anilin werden in 45 ml Dichlormethan suspendiert und auf 0°C gekühlt. Man tropft 2.57 ml (18.4 mmol) Triethylamin und 0.87 ml (12.3 mmol) Acetylchlorid hinzu und lässt noch 2.5 Stunden bei 0°C rühren. Man gibt weitere 0.86 ml (6.14 mmol) Triethylamin und 0.44 ml (6.14 mmol) Acetylchlorid hinzu und lässt eine weitere Stunde reagieren. Dann gibt man gesättigte Natriumhydrogencarbonatlösung zu und lässt 10 min bei RT rühren. Man extrahiert zweimal mit Dichlormethan. Die vereinigten organischen Phasen werden über Magnesiumsulfat getrocknet und das Lösungsmittel im Vakuum entfernt. Das Rohprodukt wird in 45 ml Ethanol aufgenommen. Man gibt 1.14 ml (6.14 mmol) 5.4M Natriummethanolatlösung hinzu und lässt 30 min bei RT rühren. Man engt ein und reinigt durch Chromatographie an Kieselgel (Eluens: Dichlormethan/Methanol (7N Ammoniak) 100:1 bis 100:5).
Ausbeute: 1.05 g (56% d. Th.)
LC-MS (Methode 2): Rₜ = 1.76 min.
MS (ESI pos.): m/z = 304 (M+H)⁺.
¹H-NMR (DMSO-d₆, 400 MHz): δ = 2.10 (s, 3H), 6.28 (dd, 1H), 6.41 (d, 1H), 7.41 (dd, 1H), 7.53 (m, 2H), 8.08 (d, 1H), 10.40 (s, 1H), 11.83 (br. s, 1H).

### Beispiel 7A

### N-{4-[(3-Brom-1H-pyrrolo[2,3-b]pyridin-4-yl)oxy]-3,5-difluorphenyl}acetamid

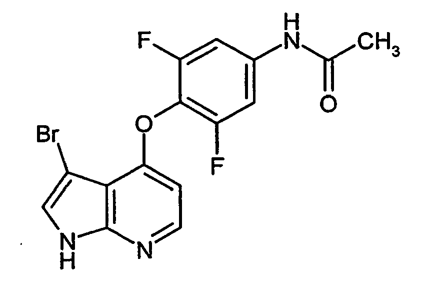

1.05 g (3.45 mmol) *N*-[3,5-Difluor-4-(1H-pyrrolo[2,3-b]pyridin-4-yloxy)phenyl]acetamid werden in 35 ml Eisessig gelöst und mit einem Eisbad auf ca. 10°C gekühlt. Man gibt 4.8 ml (4.8 mmol) einer 1M Lösung von Brom in Dichlormethan hinzu und lässt ca. 30 min bei 20°C rühren, wobei ein Feststoff ausfällt. Man saugt ab und wäscht mit Essigsäureethylester nach. Die Mutterlauge wird mit konz. Natronlauge neutralisiert und mit Essigsäureethylester extrahiert. Die abgesaugten Kristalle werden in 3 ml DMF gelöst. Man verdünnt mit Essigsäureethylester und extrahiert dreimal mit 1N Natriumhydroxidlösung. Die vereinigten organischen Phasen werden über Magnesiumsulfat getrocknet. Das Lösungsmittel wird im Vakuum entfernt. Das Produkt wird ohne weitere Aufreinigung umgesetzt.
Ausbeute: 1.32 g (100% d. Th.)
LC-MS (Methode 2): Rₜ = 2.10 min.
MS (ESI pos.): m/z= 382, 384 (M+H)⁺.
¹H-NMR (DMSO-d₆, 400 MHz): δ = 2.10 (s, 3H), 6.33 (d, 1H), 7.55 (d, 2H), 7.65 (d, 1H), 8.10 (d, 1H), 10.43 (s, 1H), 12.22 (br. s, 1H).

### Beispiel 8A

### N-[4-({3-Brom-1-[(4-methylphenyl)sulfonyl]-1H-pyrrolo[2,3-b]pyridin-4-yl}oxy)-3,5-difluorphenyl]acetamid

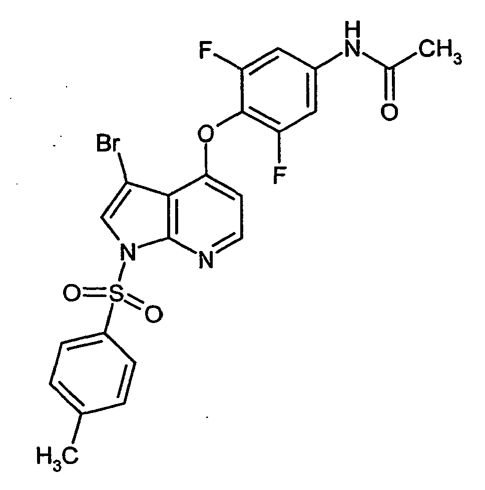

1.37 g (3.58 mmol) *N*-{4-[(3-Brom-1H-pyrrolo[2,3-b]pyridin-4-yl)oxy]-3,5-difluorphenyl}-acetamid werden in 200 ml THF gelöst und auf -78°C gekühlt. Man tropft 1.6 ml (4:0 mmol) einer 2.5M n-Butyllithiumlösung hinzu und lässt 15 min rühren. Dann werden 752 mg (3.94 mmol) p-Toluolsulfonsäurechlorid als Lösung in 7.5 ml THF zugetropft. Man lässt die Reaktionslösung auf RT erwärmen und eine Stunde rühren. Dann wird zwischen Essigsäureethylester und 0.1N Natronlauge verteilt. Die wässrige Phase wird zweimal mit Essigsäureethylester extrahiert. Die vereinigten organischen Phasen werden über Magnesiumsulfat getrocknet und das Lösungsmittel im Vakuum entfernt. Man reinigt durch Säulenchromatographie an Kieselgel (Eluens: Dichlormethan/Aceton 20:1 bis 10:1).
Ausbeute: 851 mg (44% d. Th.)
LC-MS (Methode 1): Rₜ = 2.61 min.
MS (ESI pos.): m/z= 536, 538 (M+H)⁺.
¹H-NMR (DMSO-d₆, 300 MHz): δ = 2.09 (s, 3H), 2.36 (s, 3H), 6.64 (d, 1H), 7.45 (d, 2H), 7.50-7.57 (m, 2H), 8.03 (d, 2H), 8.13 (s, 1H), 8.25 (d, 1H), 10.40 (s, 1H).

### Beispiel 9A

### N-[3,5-Difluor-4-({3-methyl-1-[(4-methylphenyl)sulfonyl]-1H-pyrrolo[2,3-b]pyridin-4-yl}oxy)-phenyl] acetamid

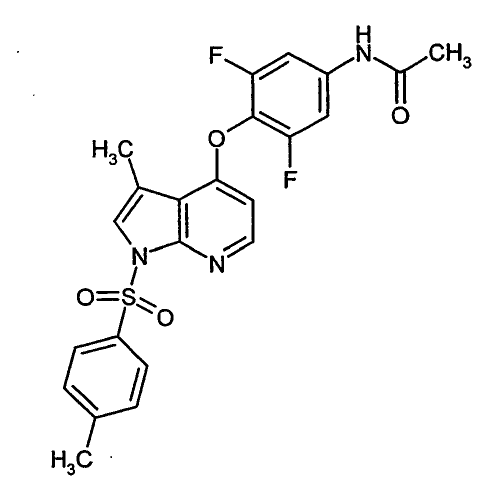

895 mg (1.67 mmol) *N*-[4-({3-Brom-1-[(4-methylphenyl)sulfonyl]-1H-pyrrolo[2,3-b]pyridin-4-yl}-oxy)-3,5-difluorphenyl]acetamid werden in Dioxan (20 ml) gelöst. Die Lösung wird entgast und mit Argon belüftet. Man gibt 2.5 ml (5.0 mmol) einer 2M Lösung von Dimethylzink in Toluol und 68 mg (0.08 mmol) [1,1'-Bis-(diphenylphosphino)ferrocen]palladium(II)chlorid-Methylen-dichlorid-Komplex hinzu und erhitzt 2 Stunden auf 100°C. Man lässt auf RT abkühlen, gibt Essigsäureethylester und 1M Salzsäure hinzu und extrahiert die wässrige Phase mit Essigsäureethylester. Die vereinigten organischen Phasen werden über Natriumsulfat getrocknet und das Lösungsmittel im Vakuum entfernt. Man reinigt den Rückstand durch Säulenchromatographie an Kieselgel (Eluens: Dichlormethan/Aceton 20:1 bis 10:1).
Ausbeute: 702 mg (89% d. Th.)
LC-MS (Methode 1): Rₜ = 2.49 min.
MS (ESI pos.): m/z = 472 (M+H)⁺.
¹H-NMR (DMSO-d₆, 400 MHz): δ = 2.09 (s, 3H), 2.35 (s, 3H), 2.41 (s, 3H), 6.52 (d, 1H), 7.42 (d, 2H), 7.52 (m, 2H), 7.66 (s, 1H), 7.97 (d, 2H), 8.17 (d, 1H), 10.43 (s, 1H).

### Beispiel 10A

### 3,5-Difluor-4-[(3-methyl-1H-pyrrolo[2,3-b]pyridin-4-yl)oxy]anilin

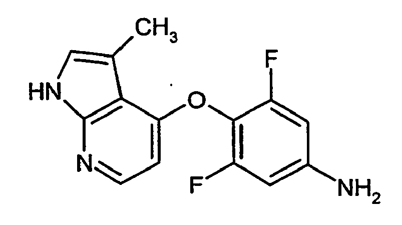

690 mg (1.46 mmol) *N*-[3,5-Difluor-4-({3-methyl-1-[(4-methylphenyl)sulfonyl]-1H-pyrrolo[2,3-b]pyridin-4-yl}oxy)phenyl]acetamid werden in 25 ml Ethanol gelöst. Man gibt 12.5 ml 20%ige Natronlauge hinzu und erhitzt das Reaktionsgemisch 4.5 Stunden auf 90°C. Die Reaktionslösung wird zwischen Essigsäureethylester und Wasser verteilt. Die wässrige Phase wird mit Essigsäureethylester extrahiert. Man trocknet die vereinigten organischen Phasen über Magnesiumsulfat und entfernt das Lösungsmittel im Vakuum. Das Produkt wird ohne weitere Aufreinigung umgesetzt.
Ausbeute: 402 mg (100% d. Th.)
LC-MS (Methode 1): Rₜ = 1.53 min.
MS (ESI pos.): m/z = 276 (M+H)⁺.
¹H-NMR (DMSO-d₆, 300 MHz): δ = 2.42 (s, 3H), 5.72 (br. s, 2H), 6.15 (d, 1H), 6.33-6.45 (m, 2H), 7.11 (s, 1H), 7.97 (d, 1H), 11.32 (br. s, 1H).

### Beispiel 11A

### 2,2,2-Trifluor-N-[3,5-difluor-4-(1H-pyrrolo[2,3-b]pyridin-4-yloxy)phenyl]acetamid

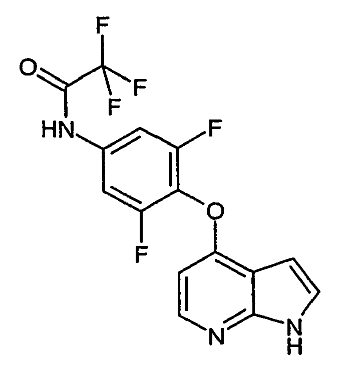

Zu einer Lösung von 200 mg (0,76 mmol) 3,5-Difluor-4-(1H-pyrrolo[2,3-b]pyridin-4-yloxy)anilin und 0.21 ml (1.53 mmol) Triethylamin in wasserfreiem Dichlormethan (20 ml) bei 0°C werden 0.16 ml (1.14 mmol) Trifluoressigsäureanhydrid hinzugetropft. Man lässt 20 min bei 0°C rühren und beendet die Reaktion durch Zutropfen einer gesättigten Natriumhydrogencarbonatlösung (10 ml). Man lässt die Suspension auf RT erwärmen und trennt die Phasen. Die wässrige Phase extrahiert man mit *tert*-Butylmethylether (10 ml). Die kombinierten organischen Phasen wäscht man mit einer gesättigten Natriumchloridlösung. Die organische Lösung wird über Magnesiumsulfat getrocknet und eingeengt. Man erhält einen Feststoff, der nicht weiter gereinigt wird.
Ausbeute: 270 mg (98% d. Th.)
HPLC (Methode 3): Rₜ = 2.21 min.
MS (ESI pos.): m/z = 358 (M+H)⁺.

### Beispiel 12A

### N-{4-[(3-Chlor-1H-pyrrolo[2,3-b]pyridin-4-yl)oxy]-3,5-difluorphenyl}-2,2,2-trifluoracetamid

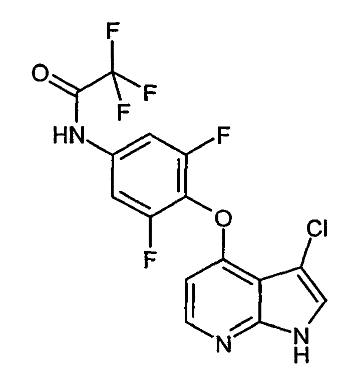

Zu einer Lösung von 250 mg (0.70 mmol) 2,2,2-Trifluor-*N*-[3,5-difluor-4-(1H-pyrrolo[2,3-b]pyridin-4-yloxy)phenyl]acetamid in wasserfreiem Tetrahydrofuran (5 ml) werden 204 mg (1.54 mmol) *N*-Chlorsuccinimid und 50 µl 1M wässrige Salzsäure zugegeben. Man lässt die Lösung bei RT über Nacht rühren. Die Titelverbindung fällt aus dem Reaktionsgemisch aus. Durch Absaugen und Trocknen erhält man einen Feststoff.
Ausbeute: 90 mg (33% d. Th.)
HPLC (Methode 3): Rₜ = 2.45 min.
MS (ESI pos.): m/z = 392, 394 (M+H)⁺.

### Beispiel 13A

### 4-[(3-Chlor-1H-pyrrolo[2,3-b]pyridin-4-yl)oxy]-3,5-difluoranilin

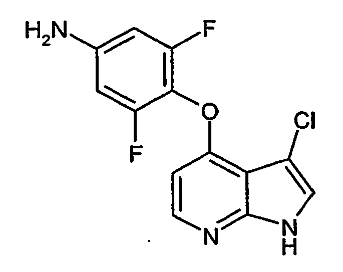

Zu einer Lösung von 90 mg (0.23 mmol) *N*-{4-[(3-Chlor-1H-pyrrolo[2,3-b]pyridin-4-yl)oxy]-3,5-difluorphenyl}-2,2,2-trifluoracetamid in Ethanol (5 ml) werden 3 ml einer 1N Natriumhydroxydlösung zugegeben. Man lässt die Reaktion über Nacht rühren. Die Lösung extrahiert man mit *tert-*Butylmethylether (zweimal 10 ml). Die kombinierten organischen Phasen wäscht man mit einer gesättigten Natriumchloridlösung. Die organische Phase wird über Magnesiumsulfat getrocknet und eingeengt. Man erhalt einen Feststoff, der nicht weiter gereinigt wird.
Ausbeute: 65 mg (96% d. Th.)
HPLC (Methode 2): Rₜ = 2.13 min.
MS (ESI pos.): m/z = 296, 298 (M+H)⁺.

### Beispiel 14A

### 4-[(6-Chlor-1H-pyrrolo[2,3-b]pyridin-4-yl)oxy]-3-fluoranilin

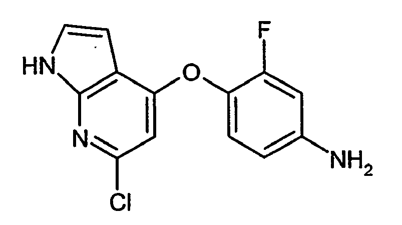

0.77 g (6.07 mmol) 4-Amino-2-fluorphenol werden in DMF gelöst. 0.68 g (6.07 mmol) Kalium-*tert*-butylat werden zugegeben und das Gemisch wird 30 Minuten bei Raumtemperatur gerührt. Dann werden nacheinander 0.35 g (2.53 mmol) gepulvertes Kaliumcarbonat und 1.00 g (5.06 mmol) 6-Chlor-4-nitro-1H-pyrrolo[2,3-b]pyridin dazugegeben. Das Gemisch wird 12 Stunden bei 120°C gerührt. Nach dem Abkühlen wird es mit Essigsäureethylester (200 ml) verdünnt. Die Suspension wird über Celite^{®} abgesaugt und mit Essigsäureethylester nachgewaschen. Die Lösung wird nacheinander mit wässriger Natriumhydrogencarbonatlösung und Natriumchloridlösung ausgeschüttelt. Die organische Phase wird über wasserfreiem Magnesiumsulfat getrocknet und eingeengt. Der Rückstand wird durch Säulenchromatographie gereinigt (Kieselgel 60, Eluens: Dichlormethan/Aceton 5 : 1).
Ausbeute: 0.95 g (67% d. Th.)

### Alternative Herstellungsmethode:

Eine Mischung aus 1.80 g, (9.11 mmol) 6-Chlor-4-nitro-1H-pyrrolo[2,3-b]pyridin, 3.78 g (27.3 mmol) Kaliumcarbonat und 3.17 g (18.2 mmol) Natriumdithionit in 26 ml DMSO wird entgast. Man setzt 2.32 g (18.2 mmol) 4-Amino-2-fluorphenol zu und erhitzt 4 Stunden auf 120°C. Dann wird mit Essigsäureethylester verdünnt und über Celite^{®} abgesaugt. Das Filtrat wird zweimal mit Natriumcarbonat und einmal mit Natriumchloridlösung gewaschen, über Natriumsulfat getrocknet und eingeengt. Man reinigt durch Säulenchromatographie an Kieselgel (Eluens: Dichlormethan/ Methanol 100 : 2).
LC-MS (Methode 2): Rₜ = 2.09 min.
MS (ESIpos): m/z = 278 (M+H)⁺.
¹H-NMR (DMSO-d₆, 200 MHz): δ = 5.52 (br. s, 2H), 6.21-6.30 (m, 2H), 6.44 (dd, 1H), 6.53 (dd, 1H), 7.07 (dd, 1H), 7.39 (dd, 1H), 11.96 (br. s, 1H).

### Beispiel 15A

### 3-Fluor-4-(1H-pyrrolo[2,3-b]pyridin-4-yloxy)anilin

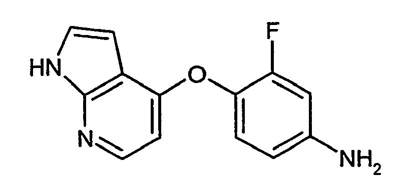

3.2 g (11.5 mmol) 4-[(6-Chlor-1H-pyrrolo[2,3-b]pyridin-4-yl)oxy]-3-fluoranilin werden in Ethanol bei 50°C gelöst. Dann lässt man die Lösung auf RT abkühlen und gibt 2.45 g (2.30 mmol) 10% Palladium auf Aktivkohle hinzu. Das Gemisch wird über Nacht unter 2 bar Wasserstoff-Druck hydriert. Anschließend wird über Kieselgur abgesaugt, mit Ethanol nachgewaschen und das Filtrat eingeengt.
Ausbeute: 2.5 g (89% d. Th.)
LC-MS (Methode 1): Rₜ =1.18 min.
MS (ESI pos.): m/z = 244 (M+H)⁺.
¹H-NMR (DMSO-d₆, 200 MHz): δ = 5.45 (mc, 2H), 6.25 (mc, 2H), 6.40-6.55 (br. 2H), 7.05 (t, 1H), 7.33 (mc, 1H), 8.25 (d, 1H), 11.69 (s, 1H).

### Beispiel 16A

### N-[3-Fluor-4-(1H-pyrrolo[2,3-b]pyridin-4-yloxy)phenyl]acetamid

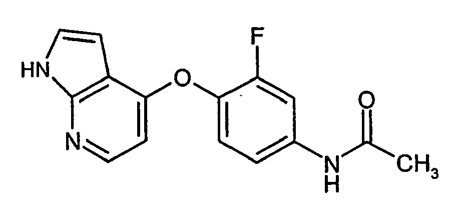

5.00 g (17.9 mmol) 3-Fluor-4-(1H-pyrrolo[2,3-b]pyridin-4-yloxy)anilin Hydrochlorid werden in 100 ml Dichlormethan suspendiert und auf 0°C gekühlt. Man tropft 9.97 ml (71.5 mmol) Triethylamin und 3.81 ml (53.6 mmol) Acetylchlorid hinzu und lässt noch 2 Stunden bei 0°C rühren. Man gibt gesättigte Natriumhydrogencarbonatlösung hinzu und lässt 10 min bei RT rühren. Dann wird zweimal mit Dichlormethan extrahiert. Unlösliche Bestandteile werden mit etwas Aceton angelöst, man verdünnt mit Dichlormethan und wäscht mit gesättigter Natriumchloridlösung. Die vereinigten organischen Phasen werden über Magnesiumsulfat getrocknet und das Lösungsmittel im Vakuum entfernt. Das Rohprodukt wird in 100 ml Ethanol aufgenommen. Man gibt 3.31 ml (17.9 mmol) 5.4 M Natriummethanolatlösung hinzu und lässt 30 min bei RT rühren. Man engt ein und reinigt durch Chromatographie an Kieselgel (Eluens: Dichlormethan/Methanol (7N Ammoniak) 100:1 bis 100:5).
Ausbeute: 3.92 g (77% d. Th.)
LC-MS (Methode 3):Rₜ= 1.56 min.
MS (ESI pos.): m/z = 286 (M+H)⁺.
¹H-NMR (DMSO-d₆, 300 MHz): δ = 2.08 (s, 3H), 6.22 (dd, 1H), 6.35 (d, 1H), 7.28-7.38 (m, 3H), 7.80 (dd, 1H), 8.07 (d, 1H), 10.21 (br. s, 1H), 11.72 (br. s, 1H).

### Beispiel 17A

### N-{4-[(3-Brom-1H-pyrrolo[2,3-b]pyridin-4-yl)oxyl-3-fluorphenyl}acetamid

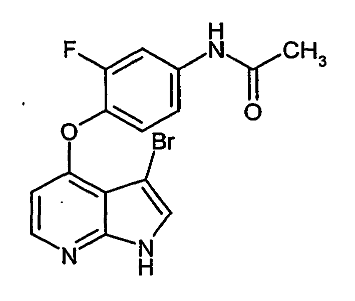

4.00 g (14.0 mmol) [3-Fluor-4-(1H-pyrrolo[2,3-b]pyridin-4-yloxy)phenyl]acetamid werden in 200 ml Eisessig gelöst und mit einem Eisbad auf ca. 10°C gekühlt. Man gibt 21.0 ml (21.0 mmol) einer 1M Lösung von Brom in Dichlormethan hinzu und lässt ca. 15 min bei 10°C rühren, wobei ein Feststoff ausfällt. Man saugt ab und wäscht mit Essigsäureethylester nach. Die abgesaugten Kristalle werden in 30 ml DMF gelöst. Man verdünnt mit 500 ml Essigsäureethylester und extrahiert dreimal mit 300 ml 1M Natriumhydroxidlösung. Man trocknet über Magnesiumsulfat und entfernt das Lösungsmittel im Vakuum.
Ausbeute: 3.44 g (66% d. Th.)
LC-MS (Methode 6): Rₜ = 2.03 min.
MS (ESI pos.): m/z= 364, 366 (M+H)⁺.
¹H-NMR (DMSO-d₆, 300 MHz): δ = 2.08 (s, 3H), 6.27 (dd, 1H), 7.28-7.38 (m, 2H), 7.60 (d, 1H), 7.82 (dd, 1H), 8.09 (d, 1H), 10.21 (br. s, 1H), 12.11 (br. s, 1H).

### Beispiel 18A

### 3-Fluor-4-({1-[(4-methylphenyl)sulfonyl]-1H-pyrrolo[2,3-b]pyridin-4-yl}oxy)anilin

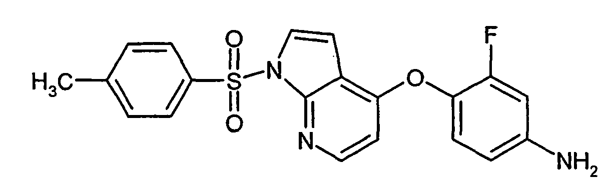

998 mg (4.10 mmol) 3-Fluor-4-(1H-pyrrolo[2,3-b]pyridin-4-yloxy)anilin werden in 50 ml THF gelöst, mit 230 mg (5.74 mmol) Natriumhydrid (in THF) versetzt und anschließend eine Stunde bei RT gerührt. Dann werden 860 mg (4.51 mmol) p-Toluolsulfonsäurechlorid dazugegeben und die Reaktionslösung für eine Stunde bei 60°C weitergerührt. Die Suspension wird über Celite^{®} filtriert und mit THF und etwas Dichlormethan/Methanol 10:1 1 nachgewaschen und das Lösungsmittel im Vakuum entfernt. Der Rückstand wird als Rohprodukt weiter umgesetzt.
Ausbeute: 1.65 g (86% d. Th.)
LC-MS (Methode 1): Rₜ = 2.39 min.

### Beispiel 19A

### N-[3-Fluor-4-({1-[(4-methylphenyl)sulfonyl]-1H-pyrrolo[2,3-b]pyridin-4-yl}oxy)phenyl]acetamid

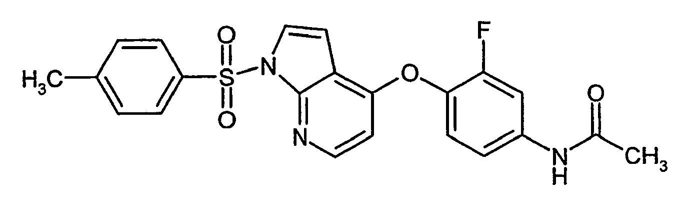

3.02 g (7.60 mmol) 3-Fluor-4-({1-[(4-methylphenyl)sulfonyl]-1H-pyrrolo[2,3-b]pyridin-4-yl}oxy)-anilin werden in 30 ml Acetanhydrid gelöst und eine Stunde bei 50°C gerührt. Dann werden flüchtige Bestandteile im Vakuum entfernt und überschüssiges Reagens mehrmals mit Toluol azeotrop entfernt. Das Rohprodukt wird über eine Kieselgelsäule aufgereinigt (Eluens: Cyclohexan/Essigsäureethylester 1:1).
Ausbeute: 2.04 g (58% d. Th.)
LC-MS (Methode 3): Rₜ = 2.50 min.
MS (ESI pos.): m/z = 440 (M+H)⁺.
¹H-NMR (DMSO-d₆, 400 MHz): δ = 2.07 (s, 3H), 2.35 (s, 3H), 6.55 (m, 1H), 6.66 (m, 1H), 7.34 (mc, 2H), 7.43 (d, 2H), 7.80 (m, 2H), 8.01 (d, 2H), 8.20 (d, 1H), 10.26 (s, 1H).

### Beispiel 20A

### N-[4-({3-Brom-1-[(4-methylphenyl)sulfonyl]-1H-pyrrolo[2,3-b]pyridin-4-yl}oxy)-3-fluorphenyl]-acetamid

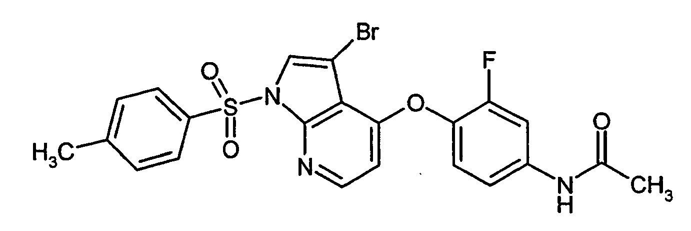

490 mg (1.11 mmol) *N*-[3-Fluor-4-({1-[(4-methylphenyl)sulfonyl]-1H-pyrrolo[2,3-b]pyridin-4-yl}-oxy)phenyl]acetamid werden in 35 ml Dichlormethan gelöst und auf 0°C gekühlt. Dazu gibt man 114 µl (2.23 mmol) Brom. Nach einer Stunde gibt man Eis hinzu sowie 10%ige Natriumthiosulfatlösung. Nach Extraktion mit Dichlormethan wird die organische Phase über Magnesiumsulfat getrocknet und das Lösungsmittel im Vakuum entfernt. Man reinigt durch Chromatographie an Kieselgel (Eluens: Dichlormethan : Aceton : 10:1).
Ausbeute: 360 mg (62% d. Th.)

### Alternative Herstellungsmethode:

3.23 g (8.87 mmol) *N*-{4-[(3-Brom-1H-pyrrolo[2,3-b]pyridin-4-yl)oxy]-3-fluorphenyl}acetamid werden in 500 ml THF gelöst und auf -78°C gekühlt. Man tropft 3.90 ml (9.76 mmol) einer 2.5M n-Butyllithiumlösung hinzu und lässt 15 min rühren. Dann werden 1.86 g (9.76 mmol) p-Toluolsulfonsäurechlorid als Lösung in 20 ml THF zugetropft. Man lässt die Reaktionslösung auf RT erwärmen und eine Stunde rühren. Dann wird Natriumhydrogencarbonatlösung zugesetzt und dreimal mit Essigsäureethylester extrahiert. Die vereinigten organischen Phasen werden über Magnesiumsulfat getrocknet und das Lösungsmittel im Vakuum entfernt.
Ausbeute: 4.22 g (92% d. Th.)
LC-MS (Methode 1): Rₜ = 2.53 min.
¹H-NMR (DMSO-d₆, 400 MHz): δ = 2.07 (s, 3H), 2.36 (s, 3H), 6.50 (m, 1H), 7.34 (m, 2H), 7.44 (d, 2H), 7.80 (m, 1H), 8.02 (d, 2H), 8.08 (s, 1H), 8.23 (d, 1H), 10.23 (s, 1H).

### Beispiel 21A

### N-[3-Fluor-4-({3-methyl-1-[(4-methylphenyl)sulfonyl]-1H-pyrrolo[2,3-b]pyridin-4-yl}oxy)-phenyl]acetamid

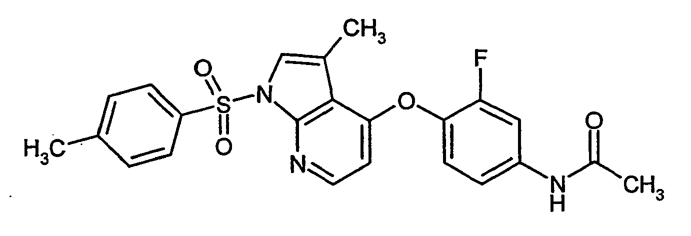

200 mg (0.39 mmol) *N*-[4-({3-Brom-1-[(4-methylphenyl)sulfonyl]-1H-pyrrolo[2,3-b]pyridin-4-yl}-oxy)-3-fluorphenyl]acetamid und 97 mg (1.16 mmol) Natriumhydrogencarbonat werden in einer Mischung aus Dimethoxyethan (10 ml) und Wasser (3 ml) suspendiert, und es wird entgast. Man gibt 15.7 mg (0.02 mmol) [1,1'-Bis-(diphenylphosphino)ferrocen]palladium(II)chlorid-Methylendichlorid-Komplex und 107 µl (0.77 mmol) Trimethylboroxin zu und erhitzt zwei Stunden auf 85°C. Zur Aufarbeitung wird das Reaktionsgemisch mit 2 ml Dichlormethan/Methanol 10:1 1 über eine Kieselgel-Extrelutkartusche filtriert und das Lösungsmittel im Vakuum entfernt. Der Rückstand wird durch präparative HPLC gereinigt.
Ausbeute: 83 mg (47% d. Th.)

### Alternative Herstellungsmethode:

100 mg (0.19 mmol) *N*-[4-({3-Brom-1-[(4-methylphenyl)sulfonyl]-1H-pyrrolo[2,3-b]pyridin-4-yl}-oxy)-3-fluorphenyl]acetamid werden in Dioxan (2.5 ml) gelöst. Die Lösung wird entgast und mit Argon belüftet. Man gibt 0.29 ml (0.58 mmol) einer 2M Lösung von Dimethylzink in Toluol und 7.9 mg (0.01 mmol) [1,1'-Bis-(diphenylphosphino)ferrocen]palladium(II)chlorid-Methylen-dichlorid-Komplex hinzu und erhitzt 2.5 Stunden auf 100°C. Man lässt auf RT abkühlen, gibt Essigsäureethylester und 1M Salzsäure hinzu und extrahiert die wässrige Phase mit Essigsäureethylester. Die vereinigten organischen Phasen werden über Natriumsulfat getrocknet und das Lösungsmittel im Vakuum entfernt. Man reinigt den Rückstand durch präparative HPLC.
Ausbeute: 77 mg (86% d. Th.)
LC-MS (Methode 3): Rₜ = 2.60 min.
MS (ESI pos.): m/z = 454 (M+H)⁺.
¹H-NMR (DMSO-d₆, 400 MHz): δ = 2.08 (s, 3H), 2.35 (s, 3H), 2.39 (s, 3H), 6.40 (d, 1H), 7.34 (m, 2H), 7.41 (d, 2H), 7.62 (m, 1H), 7.78 (m, 1H), 7.95 (d, 2H), 8.14 (d, 1H), 10.22 (s, 1H).

### Beispiel 22A

### 3-Methyl-1H-pyrrolo[2,3-b]pyridin-7-oxid

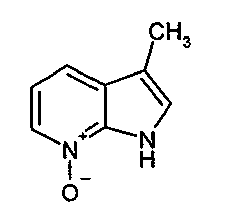

Analog zu 1H-Pyrrolo[2,3-b]pyridin-7-oxid wird die Titelverbindung durch Oxidation von 11.0 g (54.1 mmol) 3-Methyl-1H-pyrrolo[2,3-b]pyridin (Hands, D.; Bishop, B.; Cameron, M.; Edwards, T.S.; Cottrell, I.F.; Wright, S.H.B.; Synthesis 1996 (7), 877-882) mit 24.2 g (108 mmol) 3-Chlorperbenzoesäure gewonnen.
Ausbeute: 5.4 g (67% d. Th.)
LC-MS (Methode 3): Rₜ = 1.19 min.
MS (ESI pos.): m/z = 149 (M+H)⁺
¹H-NMR (DMSO-d₆, 300 MHz): δ = 2.25 (m, 3H), 7.05 (m, 1H), 7.21 (s, 1H), 7.59 (m, 1H), 8.10 (s, 1H), 12.06 (s, 1H).

### Beispiel 23A

### 4-Chlor-3-methyl-1H-pyrrolo[2,3-b]pyridin

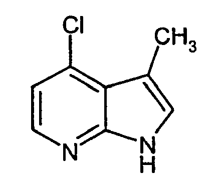

1.00 g (6.75 mmol) 3-Methyl-1H-pyrrolo[2,3-b]pyridin-7-oxid werden in 5 ml Phosphorylchlorid suspendiert. Dann werden 2 ml Chloroform dazugegeben, und das Gemisch wird über Nacht auf Rückflusstemperatur erhitzt. Man lässt auf RT abkühlen und gießt auf Essigsäureethylester/ Eiswasser. Anschließend gibt man festes Natriumcarbonat dazu. Die Phasen werden getrennt und die wässrige Phase mit Essigsäureethylester gewaschen. Die organischen Phasen werden über Natriumsulfat getrocknet und eingeengt. Der Rückstand wird durch Säulenchromatographie gereinigt (Kieselgel 60, Eluens: Cyclohexan/Methanol = 4:1).
Ausbeute: 200 mg (18% d. Th.)
LC-MS (Methode 3): Rₜ = 2.05 min.
¹H-NMR (DMSO-d₆, 200 MHz): δ = 2.41 (m, 3H), 7.10 (d, 1H), 7.31 (s, 1H), 8.07 (d, 1H), 12.44 (s, 1H).

### Beispiel 24A

### 4-Chlor-3-methyl-1H-pyrrolo[2,3-b]pyridin-7-oxid

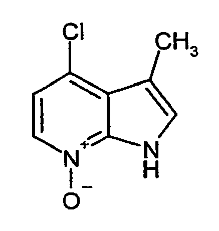

Analog zu 3-Methyl-1H-pyrrolo[2,3-b]pyridin-7-oxid wird die Titelverbindung durch Oxidation von 898 mg (5.39 mmol) 4-Chlor-3-methyl-1H-pyrrolo[2,3-b]pyridin (aus Beispiel 23A) mit 2.42 g (10.78 mmol) 3-Chlorperbenzoesäure gewonnen.
Ausbeute: 688 mg (70% d. Th.)
LC-MS (Methode 3): Rₜ = 1.75 min.
MS (ESI pos.): m/z = 183 (M+H)⁺
¹H-NMR (DMSO-d₆, 200 MHz): δ = 2.41 (m, 3H), 7.10 (d, 1H), 7.30 (s, 1H), 8.07 (d, 1H), 12.44 (s, 1H).

### Beispiel 25A

### Methyl-4,6-dichlor-3-methyl-1H-pyrrolo[2,3-b]pyridin-1-carboxylat

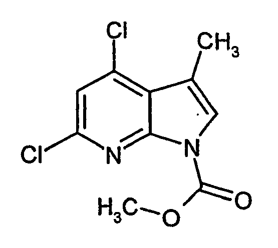

Analog zu 6-Chlor-4-nitro-1H-pyrrolo[2,3-b]pyridin wird die Titelverbindung aus 688 mg (3.77 mmol) 4-Chlor-3-methyl-1H-pyrrolo[2,3-b]pyridin-7-oxid und 1.78 g (18.84 mmol) Chlorameisen-säuremethylester und 0.61 g (3.77 mmol) Hexamethyldisilazan gewonnen.
Ausbeute: 215 mg (22% d. Th.)
LC-MS (Methode 3): Rₜ = 2.44 min.
MS (ESI pos.): m/z = 259 (M+H)⁺.
¹H-NMR (DMSO-d₆, 200 MHz): δ = 2.40 (m, 3H), 3.99 (s, 3H), 7.61 (s, 1H), 7.77 (d, 1H).

### Beispiel 26A

### 4-[(6-Chlor-3-methyl-1H-pyrrolo[2,3-b]pyridin-4-yl)oxy]-3-fluoranilin

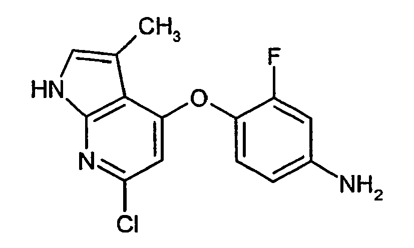

300 mg (1.16 mmol) Methyl-4,6-dichlor-3-methyl-1H-pyrrolo[2,3-b]pyridin-1-carboxylat und 320 mg (2.32 mmol) pulverisiertes Kaliumcarbonat werden in 9 ml DMSO suspendiert. Die Mischung wird entgast und 442 mg (3.48 mmol) 4-Amino-2-fluorphenol werden zugesetzt. Man erhitzt 4 Stunden auf 120°C. Nach Zugabe von Essigsäureethylester wird über Celite^{®} abgesaugt und mit Essigsäureethylester nachgewaschen. Das Filtrat wird dreimal mit gesättigter Natriumhydrogencarbonatlösung und mit gesättigter Natriumchloridlösung geschüttelt. Man trocknet über Natriumsulfat und entfernt das Lösungsmittel im Vakuum. Der Rückstand wird durch Säulenchromatographie gereinigt (Kieselgel 60, Eluens: Dichlormethan/Methanol = 50 : 1).
Ausbeute: 142 mg (42% d. Th.)
LC-MS (Methode 3): Rₜ = 2.32 min.
MS (ESI pos.): m/z = 292 (M+H)⁺.

### Beispiel 27A

### 3-Fluor-4-[(3-methyl-1H-pyrrolo[2,3-b]pyridin-4-yl)oxy]anilin

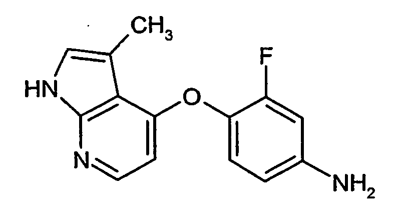

Analog zu 3-Fluor-4-(1H-pyrrolo[2,3-b]pyridin-4-yloxy)anilin wird die Titelverbindung durch katalytische Hydrierung von 142 mg (0.49 mmol) 4-[(6-Chlor-3-methyl-1H-pyrrolo[2,3-b]pyridin-4-yl)oxy]-3-fluoranilin gewonnen.
Ausbeute: 125 mg (100% d.Th.)

### Alternative Herstellungsmethode:

267 mg (0.59 mmol) *N*-[3-Fluor-4-({3-methyl-1-[(4-methylphenyl)sulfonyl]-1H-pyrrolo[2,3-b]-pyridin-4-yl}oxy)phenyl]acetamid werden in 10 ml Ethanol gelöst. Man gibt 5 ml 20%ige Natronlauge hinzu und erhitzt das Reaktionsgemisch über Nacht auf 90°C. Das Lösungsmittel wird weitgehend im Vakuum entfernt. Der Rückstand wird in Essigsäureethylester aufgenommen und mit Natriumcarbonatlösung geschüttelt. Die organische Phase wird mit Natriumchloridlösung gewaschen, über Magnesiumsulfat getrocknet und das Lösungsmittel im Vakuum entfernt.
Ausbeute: 170 mg (97% d. Th.)
LC-MS (Methode 3): Rₜ = 1.52 min.
¹H-NMR (DMSO-d₆, 300 MHz): δ = 2.41 (s, 3H), 5.38 (s, 2H), 6.08 (d, 1H), 6.40-6.56 (m, 2H), 7.00 (t, 1H), 7.08 (s, 1H), 7.93 (d, 1H), 11.26 (s, 1H).

### Beispiel 28A

### 2,2,2-Trifluor-N-[3-fluor-4-(1H-pyrrolo[2,3-b]pyridin-4-yloxy)phenyl]acetamid

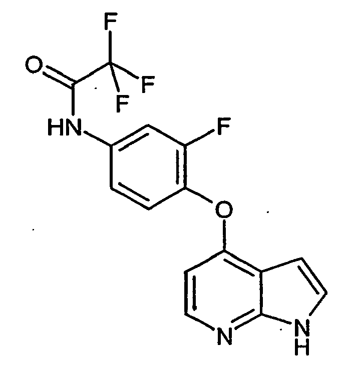

Zu einer Lösung von 650 mg (2.34 mmol) 3-Fluor-4-(1H-pyrrolo[2,3-b]pyridin-4-yloxy)anilin und 0.65 ml (4.64 mmol) Triethylamin in wasserfreiem Dichlormethan (40 ml) bei 0°C werden 0.5 ml Trifluoressigsäureanhydrid (3.48 mmol) hinzugetropft. Man lässt 20 min bei 0°C rühren und beendet die Reaktion durch Zutropfen einer gesättigten Natriumhydrogencarbonatlösung (20 ml). Man lässt die Suspension auf RT kommen und trennt die Phasen. Die wässrige Phase extrahiert man mit Essigsäureethylester (20 ml). Die kombinierten organischen Phasen wäscht man mit einer gesättigten Natriumchloridlösung. Die organische Lösung wird über Magnesiumsulfat getrocknet und eingeengt. Man erhält einen Feststoff, der nicht weiter gereinigt wird.
Ausbeute: 830 mg (96% d. Th.)
HPLC (Methode 3): Rₜ = 2.15 min.
MS (ESI pos.): m/z = 340 (M+H)⁺.
¹H-NMR (DMSO-d₆, 400 MHz): δ = 6.24 (m, 1H), 6.40 (d, 1H), 7.39 (dd, 1H), 7.46 (t, 1H), 7.59 (m, 1H), 7.82 (dd, 1H), 8.09 (d, 1H), 11.57 (s, 1H), 11.82 (br. s, 1H).

### Bespiel 29A

### N-{4-[(3-Chlor-1H-pyrrolo[2,3-b]pyridin-4-yl)oxy]-3-fluorphenyl}-2,2,2-trifluoracetamid

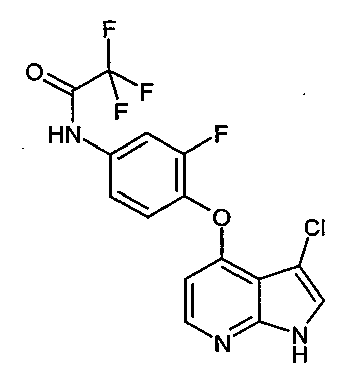

Zu einer Lösung von 540 mg (1.59 mmol) 2,2,2-Trifluor-*N*-[3-fluor-4-(1H-pyrrolo[2,3-b]pyridin-4-yloxy)phenyl]acetamid in wasserfreiem Tetrahydrofuran (20 ml) werden 233 mg (1.75 mmol) N-Chlorsuccinimid zugegeben. Man lässt die Lösung über Nacht rühren. Man gibt 20 ml einer gesättigten Natriumhydrogencarbonatlösung zu und extrahiert mit Essigsäureethylester (zweimal 20 ml). Die kombinierten organischen Phasen wäscht man mit einer gesättigten Natriumchloridlösung. Die organische Phase wird über Magnesiumsulfat getrocknet und eingeengt. Man erhält einen Feststoff, der nicht weiter gereinigt wird.
Ausbeute: 639 mg (quantitativ)
HPLC (Methode 1): Rₜ = 2.20 min.
MS (ESI pos.): m/z= 374, 376 (M+H)⁺.
¹H-NMR (DMSO-d₆, 300 MHz): δ = 6.34 (d, 1H), 7.43 (t, 1H), 7.59 (m, 2H), 7.83 (dd, 1H), 8.12 (d, 1H), 11.54 (s, 1H), 12.12 (br. s, 1H).

### Beispiel 30A

### 4-[(3-Chlor-1H-pyrrolo[2,3-b]pyridin-4-yl)oxy]-3-fluoranilin

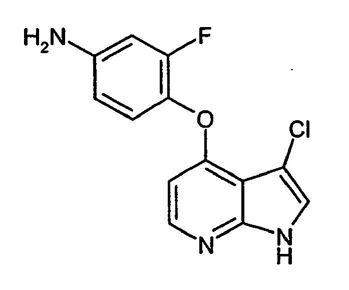

Zu einer Lösung von 637 mg (1.70 mmol) *N*-{4-[(3-Chlor-1H-pyrrolo[2,3-b]pyridin-4-yl)oxy]-3-fluorphenyl}-2,2,2-trifluoracetamid in Tetrahydrofuran (10 ml) werden 8 ml 1N Natronlauge zugegeben. Man lässt die Reaktion über Nacht rühren. Die Lösung extrahiert man mit Essigsäureethylester (zweimal 20 ml). Die kombinierten organischen Phasen wäscht man mit einer gesättigten Natriumchloridlösung. Die organische Phase wird über Magnesiumsulfat getrocknet und eingeengt. Man erhält einen Feststoff, der nicht weiter gereinigt wird.
Ausbeute: 396 mg (81% d. Th.)
HPLC (Methode 1): Rₜ = 1.78 min.
MS (ESI pos.): m/z = 278, 280 (M+H)⁺.

### Beispiel 31A

### N-[4-({3-Cyclopropyl-1-[(4-methylphenyl)sulfonyl]-1H-pyrrolo[2,3-b]pyridin-4-yl}oxy)-3-fluorphenyl] acetamid

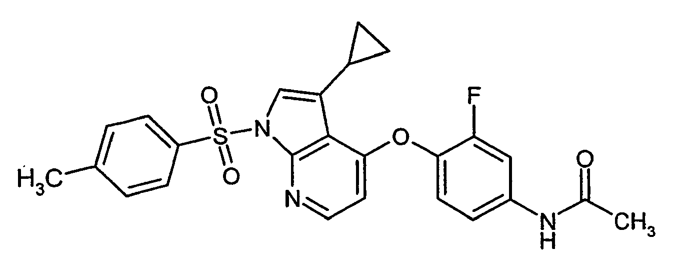

500 mg (0.96 mmol) *N*-[4-({3-Brom-1-[(4-methylphenyl)sulfonyl]-1H-pyrrolo[2,3-b]pyridin-4-yl}oxy)-3-fluorphenyl]acetamid und 400 mg (2.89 mmol) Kaliumcarbonat werden in DMF (5 ml) suspendiert. Die Lösung wird entgast und mit Argon belüftet. Dann werden -39 mg (0.05 mmol) [1,1'-Bis-(diphenylphosphino)ferrocen]palladium(II)chlorid-Methylendichlorid-Komplex und 207 mg (2.41 mmol) Cyclopropylboronsäure (Wallace, Debra J.; Chen, Cheng-yi; Tetrahedron Lett. 2002, 43(39), 6987-6990) zugegeben. Über Nacht erhitzt man auf 110°C. Dann wird die Reaktionsmischung in Dichlormethan/Methanol 10:1 gelöst und durch eine Extrelutkartusche filtriert. Man engt im Vakuum ein und reinigt das Rohprodukt durch präparative HPLC.
Ausbeute: 194 mg (39% d. Th.)
LC-MS (Methode 1): Rₜ = 2.54 min.
MS (ESI pos.): m/z = 480 (M+H)⁺.
¹H-NMR (DMSO-d₆, 400 MHz): δ = 0.73-0.78 (m, 2H), 0.85-0.91 (m, 2H), 2.07 (s, 3H), 2.15 (tt, 1H), 2.34 (s, 3H), 6.43 (d, 1H), 7.32-7.37 (m, 2H), 7.41 (d, 2H), 7.46 (s, 1H), 7.78-7.84 (m, 1H), 7.96 (d, 2H), 8.14 (d, 1H), 10.24 (br. s, 1H).

### Beispiel 32A

### 4-[(3-Cyclopropyl-1H-pyrrolo[2,3-b]pyridin-4-yl)oxy]-3-fluoranilin

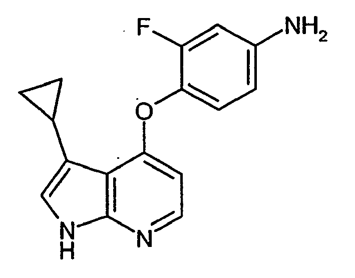

192 mg (0.40 mmol) *N*-[4-({3-Cyclopropyl-1-[(4-methylphenyl)sulfonyl]-1H-pyrrolo[2,3-b]-pyridin-4-yl}oxy)-3-fluorphenyl]acetamid werden in 7 ml Ethanol gelöst. Man setzt 3.5 ml 20%ige Natronauge hinzu und rührt das Reaktionsgemisch über Nacht bei 90°C. Das Reaktionsgemisch wird zwischen Essigsäureethylester und Wasser verteilt. Die wässrige Phase wird mit Essigsäureethylester extrahiert. Die vereinigten organischen Phasen werden über Natriumsulfat getrocknet und das Lösungsmittel im Vakuum entfernt. Das Rohprodukt wird ohne weitere Aufreinigung umgesetzt.
Ausbeute: 99 mg (87% d.Th.)
LC-MS (Methode 1): Rₜ = 1.57 min.
MS (ESI pos.): m/z = 284 (M+H)⁺.
¹H-NMR (DMSO-d₆, 300 MHz): δ = 0.57-0.64 (m, 2H), 0.77-0.85 (m, 2H), 2.12-2.23 (m, 1H), 5.37 (br. s, 2H), 6.11 (d, 1H), 6.44 (dd, 1H), 6.52 (dd, 1H), 6.95-7.05 (m, 2H), 7.94 (d, 1H), 11.28 (br. s, 1H).

### Beispiel 33A

### N-[3-Fluor-4-({1-[(4-methylphenyl)sulfonyl]-3-vinyl-1H-pyrrolo[2,3-b]pyridin-4-yl}oxy)phenyl]-acetamid

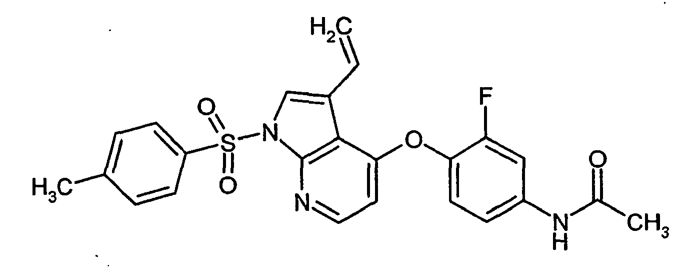

1.00 g (1.93 mmol) *N*-[4-({3-Brom-1-[(4-methylphenyl)sulfonyl]-1H-pyrrolo[2,3-b]pyridin-4-yl} - oxy)-3-fluorphenyl]acetamid und 800 mg (5.79 mmol) Kaliumcarbonat werden in DMF (6 ml) suspendiert. Die Lösung wird entgast und mit Argon belüftet. Dann werden 79 mg (0.10 mmol) [1,1'-Bis-(diphenylphosphino)ferrocen]palladium(II)chlorid-Methylendichlorid-Komplex und 0.85 ml (3.86 mmol) Vinylboronsäuredi-n-butylester zugegeben, und es wird 3 Stunden bei 90°C gerührt. Nach Zugabe von Natriumhydrogencarbonatlösung wird dreimal mit Dichlormethan extrahiert. Die vereinigten organischen Phasen werden eingeengt, und das Rohprodukt wird durch Chromatographie an Kieselgel (Eluens: Dichlormethan/Aceton 20:1 bis 10:1) gereinigt.
Ausbeute: 801 mg (89% d. Th.)
LC-MS (Methode 3): Rₜ = 2.69 min.
MS (ESI pos.): m/z = 466 (M+H)⁺.
¹H-NMR (DMSO-d₆, 400 MHz): δ = 2.07 (s, 3H), 2.35 (s, 3H), 5.31 (d, 1H), 5.98 (d, 1H), 6.45 (d, 1H), 7.00 (dd, 1H), 7.33-7.40 (m, 2H), 7.44 (d, 2H), 7.79-7.85 (m, 1H), 8.02 (d, 2H), 8.14 (s, 1H), 8.18 (d, 1H), 10.27 (br. s, 1H).

### Beispiel 34A

### N-[4-({3-Ethyl-1-[(4-methylphenyl)sulfonyl]-1H-pyrrolo[2,3-b]pyridin-4-yl}oxy)-3-fluorphenyl]-acetamid

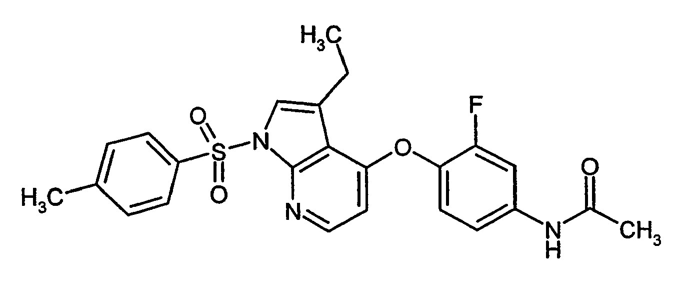

82 mg (0.18 mmol) *N*-[3-Fluor-4-({1-[(4-methylphenyl)sulfonyl]-3-vinyl-1H-pyrrolo[2,3-b]-pyridin-4-yl}oxy)phenyl]acetamid werden in 25 ml Ethanol gelöst. Man setzt 19 mg (0.02 mmol) 10%iges Palladium auf Kohle hinzu und hydriert über Nacht bei 1.5 atm Wasserstoffatmosphäre. Danach wird über Celite^{®} filtriert und das Lösungsmittel im Vakuum entfernt. Das Produkt wird ohne weitere Aufreinigung weiter umgesetzt.
Ausbeute: 60 mg (73% d. Th.)
LC-MS (Methode 1): Rₜ = 2.54 min.
MS (ESI pos.): m/z = 468 (M+H)⁺.
¹H-NMR (DMSO-d₆, 300 MHz): δ = 1.27 (t, 3H), 2.07 (s, 3H), 2.35 (s, 3H), 2.81 (dq, 2H), 6.40 (dd, 1H), 7.33-7.36 (m, 2H), 7.41 (d, 2H), 7.58 (s, 1H), 7.77-7.84 (m, 1H), 7.98 (d, 2H), 8.14 (d, 1H), 10.24 (br. s, 1H).

### Beispiel 35A

### 4-[(3-Ethyl-1H-pyrrolo[2,3-b]pyridin-4-yl)oxy]-3-fluoranilin

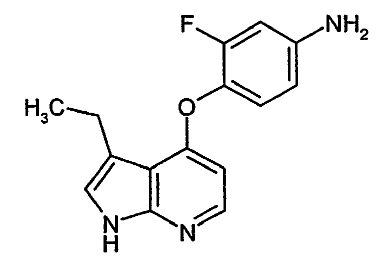

50 mg (0.11 mml) *N*-[4-({3-Ethyl-1-[(4-methylphenyl)sulfonyl]-1H-pyrrolo[2,3-b]pyridin-4-yl}-oxy)-3-fluorphenyl]acetamid werden in 3 ml Ethanol gelöst. Man setzt 1 ml 1N Natronlauge hinzu und lässt über Nacht bei 90°C rühren. Die Reaktionslösung wird zwischen Essigsäureethylester und gesättigter Natriumhydrogencarbonatlösung verteilt. Die wässrige Phase wird mit Essigsäureethylester extrahiert. Die vereinigten organischen Phasen werden über Magnesiumsulfat getrocknet und eingeengt. Das erhaltene Rohprodukt wird ohne weitere Aufreinigung umgesetzt.
Ausbeute: 24.8 mg (85% d. Th.)
LC-MS (Methode 1): Rₜ = 1.53 min.
MS (ESI pos.): m/z = 272 (M+H)⁺.

### Beispiel 36A

### N-[3-Fluor-4-({3-(2-hydroxyethyl)-1-[(4-methylphenyl)sulfonyl]-1H-pyrrolo[2,3-b]pyridin-4-yl}oxy)phenyl]acetamid

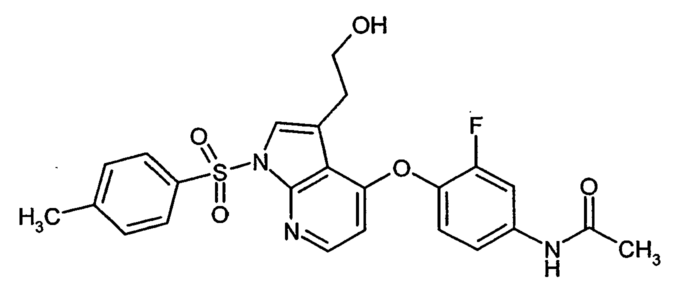

890 mg (1.91 mmol) *N*-[3-Fluor-4-({1-[(4-methylphenyl)sulfonyl]-3-vinyl-1H-pyrrolo[2,3-b]-pyridin-4-yl}oxy)phenyl]acetamid werden in 28 ml THF bei 0°C vorgelegt. Man setzt tropfenweise 7.65 ml (7.65 mmol) einer 1M Lösung von Boran in THF zu und lässt eine Stunde bei RT rühren. Das Reaktionsgemisch wird vorsichtig mit 14 ml 1N Natronlauge und 14 ml 30%iger Wasserstoffperoxydlösung versetzt. Dann wird eine Stunde lang auf 60°C erwärmt. Zur Aufarbeitung wird die Reaktionslösung zwischen Essigsäureethylester und Natriumhydrogen-carbonatlösung verteilt. Man extrahiert mit Essigsäureethylester und trocknet die vereinigten organischen Phasen über Natriumsulfat. Das Lösungsmittel wird im Vakuum entfernt. Man reinigt durch Chromatographie an Kieselgel (Eluens: Dichlormethan/Aceton 20:1 bis 6.5 : 1).
Ausbeute: 473 mg (51% d. Th.)
LC-MS (Methode 1): Rₜ = 2.00 min.
MS (ESI pos.): m/z = 484 (M+H)⁺.
¹H-NMR (DMSO-d₆, 400 MHz): δ = 2.07 (s, 3H), 2.35 (s, 3H), 2.95 (t, 2H), 3.72 (q, 2H), 4.69 (t, 1H), 6.39 (d, 1H), 7.32-7.39 (m, 2H), 7.42 (d, 2H), 7.63 (s, 1H), 7.79-7.83 (m, 1H), 7.98 (d, 2H), 8.14 (d, 1H), 10.26 (br. s, 1H).

### Beispiel 37A

### 2-[4-(4-Amino-2-fluorphenoxy)-1H-pyrrolo[2,3-b]pyridin-3-yl]ethanol

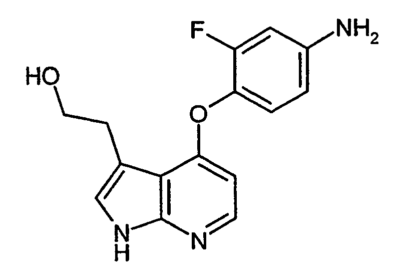

470 mg (0.97 mmol) *N*-[3-Fluor-4-({3-(2-hydroxyethyl)-1-[(4-methylphenyl)sulfonyl]-1H-pyrrolo-[2,3-b]pyridin-4-yl}oxy)phenyl]acetamid werden in 25 ml Ethanol gelöst. Man setzt 10 ml 20%ige Natronlauge hinzu und rührt das Reaktionsgemisch über Nacht bei 90°C. Das Lösungsmittel wird weitgehend im Vakuum entfernt, der Rückstand in Essigsäureethylester aufgenommen und zwischen Essigsäureethylester und 1N Natronlauge verteilt und extrahiert. Die organische Phase wird mit 1N Natronlauge gewaschen, über Magnesiumsulfat getrocknet und das Lösungsmittel im Vakuum entfernt.
Ausbeute: 248 mg (89% d. Th.)
LC-MS (Methode 6): Rₜ = 1.13 min.
MS (ESI pos.): m/z = 288 (M+H)⁺.
¹H-NMR (DMSO-d₆, 400 MHz): δ = 2.95 (t, 2H), 3.69 (q, 2H), 4.55 (t, 1H), 5.43 (br. s, 2H), 6.08 (d, 1H), 6.44 (dd, 1H), 6.52 (dd, 1H), 7.01 (t, 1H), 7.13 (d, 1H), 7.94 (d, 1H), 11.37 (br. s, 1H).

### Beispiel 38A

### 2-{4-[4-(Acetylamino)-2-fluorphenoxy]-1-[(4-methylphenyl)sulfonyl]-1H-pyrrolo[2,3-b]pyridin-3-yl}ethyl 4-methylbenzolsulfonat

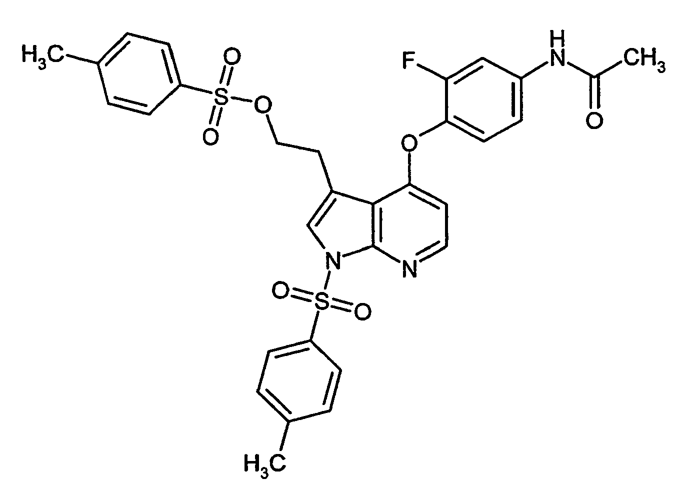

50 mg (0.10 mmol) *N*-[3-Fluor-4-({3-(2-hydroxyethyl)-1-[(4-methylphenyl)sulfonyl]-1H-pyrrolo-[2,3-b]pyridin-4-yl}oxy)phenyl]acetamid werden in 2.0 ml Dichlormethan vorgelegt. Man setzt 25 µl (0.31 mmol) Pyridin und 19.7 mg (0.10 mmol) p-Toluolsulfonsäurechlorid hinzu und lässt bei RT über Nacht rühren. Danach werden im 2-Stunden-Abstand noch zweimal die gleichen Mengen Pyridin und p-Toluolsulfonsäurechlorid zugesetzt. Nach vollständiger Umsetzung wird die Reaktionslösung mit Essigsäureethylester verdünnt und zweimal mit gesättigter Ammoniumchloridlösung extrahiert. Die organische Phase wird über Magnesiumsulfat getrocknet und das Lösungsmittel im Vakuum entfernt. Das Rohprodukt wird mittels präparativer HPLC aufgereinigt.
Ausbeute: 26 mg (38% d. Th.)
LC-MS (Methode 3): Rₜ = 2.68 min.
MS (ESI pos.): m/z = 638 (M+H)⁺.
¹H-NMR (DMSO-d₆, 300 MHz): δ = 2.08 (s, 3H), 2.20 (s, 3H), 2.35 (s, 3H), 3.08 (t, 2H), 4.35 (t, 2H), 6.28 (dd, 1H), 6.97 (d, 2H), 7.22 (t, 1H), 7.33-7.39 (m, 1H), 7.40-7.47 (m, 4H), 7.65 (s, 1H), 7.79 (dd, 1H), 8.01 (d, 2H), 8.11 (d, 1H), 10.24 (br. s, 1H).

### Beispiel 39A

### N-(3-Fluor-4-{[3-(2-methoxyethyl)-1H-pyrrolo[2,3-b]pyridin-4-yl]oxy}phenyl)acetamid

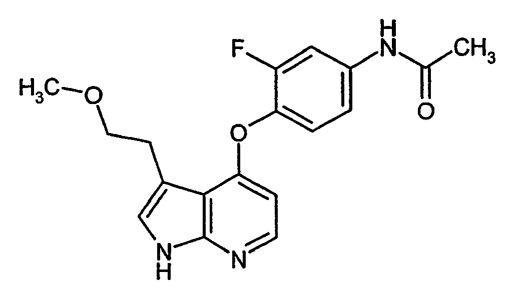

150 mg (0.24 mmol) 2-{4-[4-(Acetylamino)-2-fluorphenoay]-1-[(4-methylphenyl)sulfonyl]-1H-pyrrolo[2,3-b]pyridin-3-yl}ethyl-4-methylbenzolsulfonat werden in 6 ml Methanol gelöst. Man gibt 0.17 ml (0.94 mmol) einer 5.4M Natriummethanolatlösung und lässt über Nacht bei 45°C rühren. Die Reaktionslösung wird zwischen 1N Natronlauge und Essigsäureethylester verteilt. Man extrahiert die wässrige Phase mit Essigsäureethylester und trocknet die vereinigten organischen Phasen über Magnesiumsulfat. Das Lösungsmittel wird im Vakuum entfernt. Das erhaltene Produkt wird ohne weitere Aufreinigung umgesetzt.
Ausbeute: 55 mg (68% d. Th.)
LC-MS (Methode 2): Rₜ = 1.57 min.
MS (ESI pos.): m/z = 344 (M+H)⁺.
¹H-NMR (DMSO-d₆, 400 MHz): δ = 2.08 (s, 3H), 3.02 (t, 2H), 3.23 (s, 3H), 3.62 (t, 2H), 6.15 (d, 1H), 7.20 (d, 1H), 7.30-7.38 (m, 2H), 7.79-7. 84 (m, 1H), 7.99 (d, 1H), 10.24 (s, 1H), 11.49 (br. s, 1H).

### Beispiel 40A

### 3-Fluor-4-{[3-(2-methoxyethyl)-1H-pyrrolo[2,3-b]pyridin-4-yl]oxy}anilin

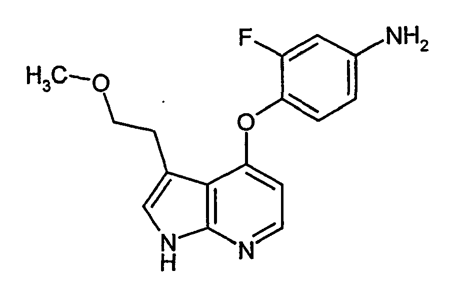

63 mg (0.18 mmol) *N*-(3-Fluor-4-{[3-(2-methoxyethyl)-1H-pyrrolo[2,3-b]pyridin-4-yl]oxy}-phenyl)acetamid werden in 5 ml Ethanol gelöst. Man setzt 2 ml 20%ige Natronlauge hinzu und lässt über Nacht bei 90°C rühren. Die Reaktionsmischung wird dann zwischen Essigsäureethylester und 1N Natronlauge verteilt. Die wässrige Lösung wird mit Essigsäureethylester extrahiert und die vereinigten organischen Phasen werden mit 1N Natronlauge gewaschen. Man trocknet über Magnesiumsulfat und entfernt das Lösungsmittel im Vakuum. Das Produkt wird ohne Aufreinigung weiter umgesetzt.
Ausbeute: 49 mg (89% d. Th.)
LC-MS (Methode 1): Rₜ = 1.27 min.
MS (ESI pos.): m/z = 302 (M+H)⁺.
¹H-NMR (DMSO-d₆, 300 MHz): δ = 3.03 (t, 2H), 3.24 (s, 3H), 3.63 (t, 2H), 5.39 (br. s, 2H), 6.10 (d, 1H), 6.44 (dd, 1H), 6.53 (dd, 1H), 7.02 (t, 1H), 7.15 (d, 1H), 7.95 (d, 1H), 11.37 (br. s, 1H).

### Beispiel 41A

### N-[4-({3-Cyano-1-[(4-methylphenyl)sulfonyl]-1H-pyrrolo[2,3-b]pyridin-4-yl}oxy)-3-fluorphenyl]-acetamid (A) und N-{4-[(3-Cyano-1H-pyrrolo[2,3-b]pyridin-4-yl)oxy]-3-fluorphenyl}acetamid (B)

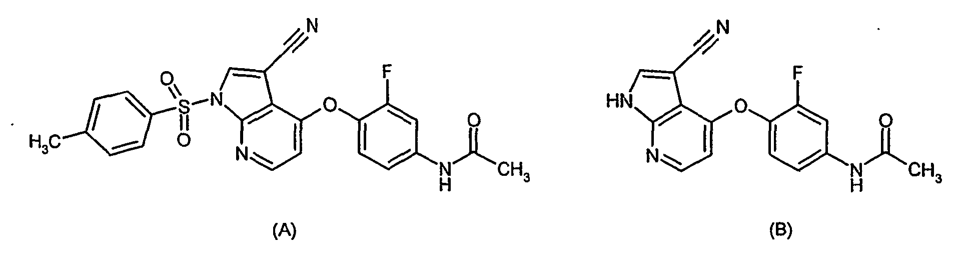

150 mg (0.29 mmol) N-[4-({3-Brom-1-[(4-methylphenyl)sulfonyl]-1H-pyrrolo[2,3-b]pyridin-4-yl}oxy)-3-fluorphenyl]acetamid, 21.2 mg (0.12 mmol) Zinkacetat, 7.6 mg (0.12 mmol) Zinkstaub, 18.4 mg (0.16 mmol) Zinkcyanid, 4.8 mg (0.087 mmol) Bis(diphenylphosphino)ferrocen und 2.65 mg (0.029 mmol) Tris(dibenzylidenaceton)dipalladium werden in einem verschraubbaren Reagenzrohr vorgelegt. Man gibt unter Argon eine entgaste Mischung aus 1 ml DMF und 0.01 ml Wasser hinzu und erhitzt im geschlossenen Gefäß 15 Stunden auf 100°C. Die Rohmischung wird durch präparative HPLC aufgereinigt. Man erhält ein Gemisch aus der tosylierten (A) und detosylierten Verbindung (B), das ohne weitere Aufreinigung umgesetzt wird.
Ausbeute: 101 mg (17% d. Th. A, 82% d. Th. B)
LC-MS (Methode 1): Rₜ = 1.47 min. (B); 2.29 min. (A)
MS (ESI pos.): m/z = 311 (M+H)⁺ (B), 465 (M+H)⁺ (A).
¹H-NMR (DMSO-d₆, 300 MHz, Verbindung A): δ = 2.08 (s, 3H), 2.38 (s, 3H), 6.61 (d, 1H), 7.35-7.44 (m, 2H), 7.48 (d, 2H), 7.83 (dd, 1H), 8.08 (d, 2H), 8.31 (d, 1H), 8.95 (s, 1H), 10.29 (s, 1H).

### Beispiel 42A

### 4-(4-Amino-2-fluorphenoxy)-1H-pyrrolo[2,3-b]pyridin-3-carbonitril

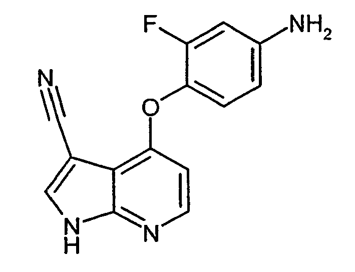

30 mg (0.065 mmol) N-[4-({3-Cyano-1-[(4-methylphenyl)sulfonyl]-1H-pyrrolo[2,3-b]pyridin-4-yl}oxy)-3-fluorphenyl]acetamid werden in einer Mischung aus 3 ml Ethanol und 3 ml THF gelöst. Man setzt 1.5 ml 20% ige Natronlauge hinzu und erhitzt über Nacht auf 90°C. Danach wird nochmals 1 ml 20%ige Natronlauge zugesetzt, ein weiterer ml nach 6 Stunden. Man lässt erneut über Nacht rühren. Dann wird die Reaktionsmischung zwischen Essigsäureethylester und Wasser verteil. Die wässrige Phase wird mit Essigsäureethylester extrahiert. Die vereinigten organischen Phasen trocknet man über Magnesiumsulfat und entfernt das Lösungsmittel im Vakuum. Das Rohprodukt wird ohne weitere Aufreinigung umgesetzt.
Ausbeute: 19 mg (100% d. Th.)
LC-MS (Methode 3): Rₜ = 1.70 min.
MS (ESI pos.): m/z = 269 (M+H)⁺.
¹H-NMR (DMSO-d₆, 300 MHz): δ = 5.48 (br. s, 2H), 6.30 (d, 1H), 6.45 (dd, 1H), 6.54 (dd, 1H), 7.07 (t, 1H), 8.14 (d, 1H), 8.31 (s, 1H), 12.7 (br. s, 1H).

### Beispiel 43A

### N-[4-({3-Cyano-1-[(4-methylphenyl)sulfonyl]-1H-pyrrolo[2,3-b]pyridin-4-yl}oxy)-3,5-difluorphenyl]acetamid (A) und N-{4-[(3-Cyano-1H-pyrrolo[2,3-b]pyridin-4-yl)oxy]-3,5-difluorphenyl}-acetamid (B)

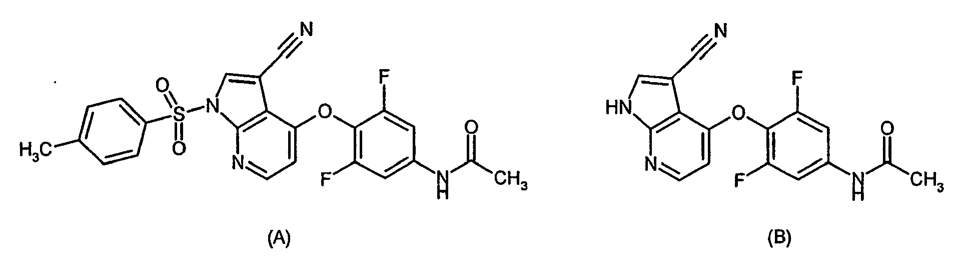

2.90 g (5.41 mmol) N-[4-({3-Brom-1-[(4-methylphenyl)sulfonyl]-1H-pyrrolo[2,3-b]pyridin-4-yl}oxy)-3,5-difluorphenyl]acetamid, 397 mg (2.16 mmol) Zinkacetat, 141 mg (2.16 mmol) Zinkstaub, 343 mg (2.92 mmol) Zinkcyanid, 89.9 mg (0.16 mmol) Bis(diphenylphosphino)ferrocen und 49.5 mg (0.05 mmol) Tris(dibenzylidenaceton)dipalladium werden vorgelegt. Man gibt unter Argon eine entgaste Mischung aus 33 ml DMF und 0.33 ml Wasser hinzu und erhitzt 20 Stunden auf 100°C. Die Rohmischung wird durch eine kurze Kieselgelsäule filtriert (Eluens: Dichlormethan:Methanol =3:1). Das erhaltene Produkt, 5 g eines Öls, das zu 37% (A) und zu 31% (B) enthält, wird ohne weitere Aufreinigung weiter umgesetzt. Eine analytische Menge wird durch präparative HPLC aufgereinigt.
LC-MS (Methode 3): Rₜ = 1.86 min. (B); 2.61 min. (A)
MS (ESI pos.): m/z = 329 (M+H)⁺ (B), 483 (M+H)⁺ (A).
¹H-NMR (DMSO-d₆, 400 MHz, Verbindung A): δ = 2.10 (s, 3H), 2.38 (s, 3H), 6.78 (d, 1H), 7.48 (d, 2H), 7.55 (d, 2H), 8.09 (d, 2H), 8.34 (d, 1H), 8.98 (s, 1H), 10.45 (s, 1H).
¹H-NMR (DMSO-d₆, 400 MHz, Verbindung B): δ = 2.11 (s, 3H), 6.54 (d, 1H), 7.57 (d, 2H), 8.23 (d, 1H), 8.45 (s, 1H), 10.45 (s, 1H), 12.99 (s, 1H).

### Beispiel 44A

### 4-(4-Amino-2,6-fluorphenoxy)-1H-pyrrolo[2,3-b]pyridin-3-carbonitril

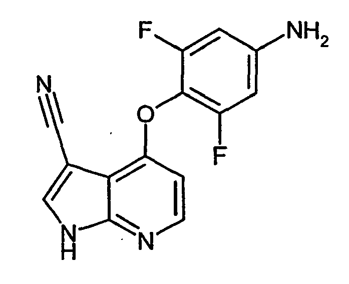

4.8 g des Rohproduktes der Umsetzung zu *N-*[4-({3-Cyano-1-[(4-methylphenyl)sulfonyl]-1H-pyrrolo[2,3-b]pyridin-4-yl}oxy)-3,5-difluorphenyl]acetamid und *N*-{4-[(3-Cyano-1H-pyrrolo[2,3-b]pyridin-4-yl)oxy]-3,5-difluorphenyl}acetamid werden in 40 ml THF und 150 ml Ethanol suspendiert. Man versetzt mit 50 ml 20%iger Natronlauge und läßt 20 h bei 90°C rühren. Nach Abkühlen engt man im Vakuum ein und versetzt mit Wasser und Essigsäureethylester. Die organische Phase wird abgetrennt und über Aktivkohle geklärt. Man trocknet über Natriumsulfat und engt ein, wobei ein kristalliner Rückstand verbleibt, der ohne weitere Aufreinigung umgesetzt wird. Eine analytische Probe wird per HPLC aufgereinigt.
Ausbeute: 1.60 g (61% d. Th.)
LC-MS (Methode 3): Rₜ = 1.89 min.
MS (ESI pos.): m/z = 287 (M+H)⁺.
¹H-NMR (DMSO-d₆, 400 MHz): δ = 5.84 (s, 2H), 6.41 (d, 2H), 6.47 (d, 1H), 8.22 (d, 1H), 8.41 (s, 1H), 12.92 (s, 1H).

### Beispiel 45A

### N-[4-({3-Cyclopropyl-1-[(4-methylphenyl)sulfonyl]-1H-pyrrolo[2,3-b]pyridin-4-yl}oxy)-3,5-difluorphenyl]acetamid

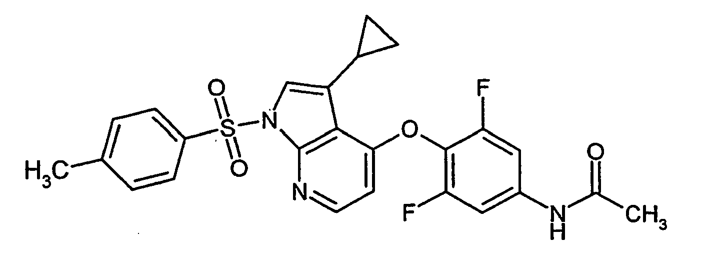

1.00 g (1.86 mmol) *N*-[4-({3-Brom-1-[(4-methylphenyl)sulfonyl]-1H-pyrrolo[2,3-b]pyridin-4-yl}-oxy)-3,5-difluorphenyl]acetamid, 1.12 g (13.1 mmol) Cyclopropylboronsäure (Wallace, Debra J.; Chen, Cheng-yi; Tetrahedron Lett. 2002, 43(39), 6987-6990), 52.3 mg (0.19 mmol) Tricyclohexylphosphin und 1.39 g (6.53 mmol) Kaliumphosphat werden in 10 ml Toluol und 0.5 ml Wasser suspendiert. Man entgast die Lösung und setzt 20.9 mg (0.09 mmol) Palladium(II)acetat hinzu. Man erhitzt 1.5 h auf 100°C. Nach dem Abkühlen versetzt man mit Essigsäureethylester, trennt die organische Phase ab und wäscht sie zweimal mit Wasser. Man trocknet über Natriumsulfat und engt ein. Man reinigt durch präparative HPLC.
Ausbeute: 666 mg (72% d. Th.)
LC-MS (Methode 1): Rₜ = 2.59 min.
MS (ESI pos.): m/z = 498 (M+H)⁺.
¹H-NMR (DMSO-d₆, 300 MHz): δ = 0.73-0.80 (m, 2H), 0.86-0.94 (m, 2H), 2.09 (s, 3H), 2.10-2.21 (m, 1H), 2.35 (s, 3H), 6.55 (d, 1H), 7.41 (d, 2H), 7.51 (s, 1H), 7.53 (d, 2H), 7.97 (d, 2H), 8.17 (d, 1H), 10.43 (s, 1H).

### Beispiel 46A

### 4-[(3-Cyclopropyl-1H-pyrrolo[2,3-b]pyridin-4-yl)oay]-3,5-difluoranilin

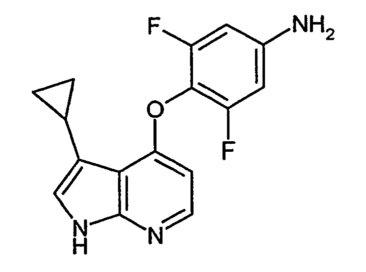

624 mg (1.25 mmol) *N*-[4-({3-Cyclopropyl-1-[(4-methylphenyl)sulfonyl]-1H-pyrrolo[2,3-b]-pyridin-4-yl}oxy)-3,5-difluorphenyl]acetamid werden in 17 ml Ethanol gelöst. Man setzt 10 ml halbkonzentrierte Natronlauge hinzu und rührt das Reaktionsgemisch 15 h bei 90°C. Das Reaktionsgemisch wird zwischen Essigsäureethylester und Wasser verteilt. Die organische Phase wird zweimal mit Wasser gewaschen, über Natriumsulfat getrocknet und im Vakuum eingeengt. Man erhält einen kristallinen Rückstand.
Ausbeute: 360 mg (92% d. Th.)
LC-MS (Methode 1): Rₜ=1.73 min.
MS (ESI pos.): m/z = 302 (M+H)⁺.
¹H-NMR (DMSO-d₆, 300 MHz): δ = 0.59-0.65 (m, 2H), 0.79-0.87 (m, 2H), 2.12-2.22 (m, 1H), 5.76 (br. s, 2H), 6.18 (d, 1H), 6.34-6.43 (m, 2H), 7.03 (d, 1H), 7.98 (d, 1H), 11.39 (s, 1H).

### Beispiel 47A

### N-{4-[(3-Brom-1-{[2-(trimethylsilyl)ethoxy]methyl}-1H-pyrrolo[2,3-b]pyridin-4-yl)oxy]-3,5-difluorphenyl}-2,2,2-trifluoracetamid

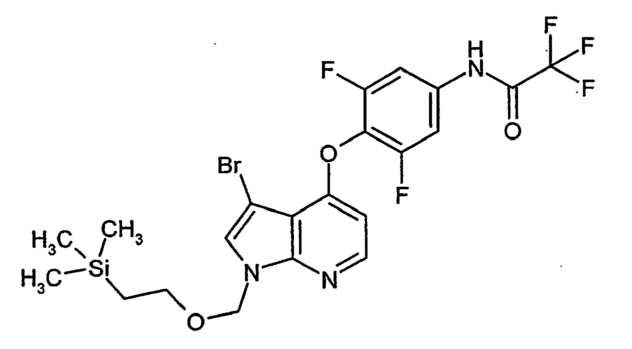

Zu einer Lösung von 215 mg *N*-{3,5-Difluor-4-[(1-{[2-(trimethylsilyl)ethoxy]methyl}-1H-pyrrolo[2,3-b]pyridin-4-yl)oxy]phenyl}-2,2,2-trifluoracetamid (440 µmol) in 10 ml Dichlormethan werden bei 0°C 485 µl einer 1M Lösung von Brom (490 µmol) in Dichlormethan getropft. Nach Rühren für 15 min bei 0°C wird die Reaktionsmischung auf eine Mischung aus 10%iger Natriumthiosulfat-Lösung und Eis gegossen, zweimal mit Essigsäureethylester extrahiert, und die vereinigten organischen Phasen werden über Magnesiumsulfat getrocknet. Nach Einengen im Vakuum wird der Rückstand mittels präparativer HPLC gereinigt.
Ausbeute: 246 mg (98% d. Th.)
LC-MS (Methode 1): Rₜ = 3.05 min.
MS (ESI pos.): m/z = 568 (M+H)⁺.
¹H-NMR (DMSO-d₆,4.00 MHz): δ = -0.01 (s, 9H), 0.82 (t, 2H), 3.53 (t, 2H), 5.60 (s, 2H), 6.48 (d, 1H), 7.70 (d, 2H), 7.88 (s, 1H), 8.17 (d, 1H), 11.72 (s, 1H).

### Beispiel 48A

### 4-[(3-Brom-1H-pyrrolo[2,3-b]pyridin-4-yl)oxy]-3,5-difluoranilin

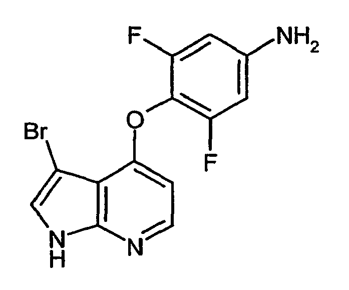

Eine Lösung von 201 mg *N*-{4-[(3-Brom-1-{[2-(trimethylsilyl)ethoxy]methyl}-1H-pyrrolo[2,3-b]pyridin-4-yl)oxy]-3,5-difluorphenyl}-2,2,2-trifluoracetamid (280 µmol) in 4.00 ml einer Mischung von Chlorwasserstoff in Dioxan (4N) wird über Nacht bei RT gerührt, die ausgefallenen Kristalle werden abfiltriert und mit Diethylether gewaschen. Der feste Rückstand (131 mg) wird in 20 ml THF gelöst. Dann werden 2.7 ml einer 5%igen Lösung von Lithiumhydroxid in Wasser (5.62 mmol) zugegeben und die Mischung wird für 60 h bei RT gerührt. Das Reaktionsgemisch wird mit Wasser verdünnt und zweimal mit Essigsäureethylester extrahiert. Nach Trocknung über Magnesiumsulfat werden die vereinigten organischen Phasen eingeengt und der Rückstand mittels präparativer HPLC gereinigt.
Ausbeute: 51 mg (51% d. Th.)
LC-MS (Methode 3): Rₜ = 2.50 min.
MS (ESI neg.): m/z= 338 (M-H)⁻.
¹H-NMR (DMSO-d₆, 400 MHz): δ = 5.79 (s, 2H), 6.28 (d, 1H), 6.39 (d, 2H), 7.58-7.63 (m, 1H), 8.08 (d, 1H), 12.12 (s, 1H).

### Ausführungsbeispiele

### Beispiel 1

### 6-Chlor-N⁴-{3,5-difluor-4-[(3-methyl-1H-pyrrolo[2,3-b]pyridin-4-yl)oxy]phenyl}pyrimidin-2,4-diamin

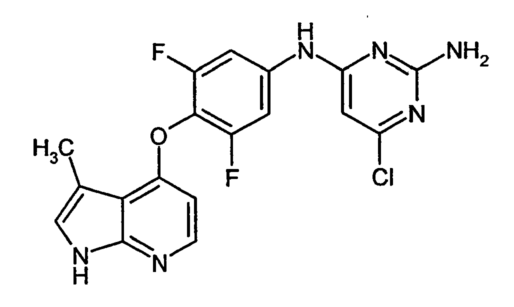

415 mg (1.51 mmol) 3,5-Difluor-4-[(3-methyl-1H-pyrrolo[2,3-b]pyridin-4-yl)oxy]anilin und 321 mg (1.96 mmol) 4,6-Dichlorpyrimidin-2-amin werden in 8 ml Wasser suspendiert. Man setzt 1.96 ml 1N Salzsäure hinzu und erhitzt über Nacht auf Rückfluss. Dann wird mit 1N Natronlauge pH10 eingestellt, wobei Kristalle ausfallen. Man saugt ab und wäscht mit Wasser nach. Das Rohprodukt wird durch Säulenchromatographie an Kieselgel (Eluens: Dichlormethan/Methanol (7N Ammoniak) 100:1 bis 100:5) gereinigt.
Ausbeute: 575 mg (95% d. Th.)
LC-MS (Methode 2): Rₜ = 2.14 min.
MS (ESI pos.): m/z = 403 (M+H)⁺.
¹H-NMR (DMSO-d₆, 300 MHz): δ = 2.44 (s, 3H), 6.04 (s, 1H), 6.20 (d, 1H), 7.00 (br. s, 2H), 7.16 (s, 1H), 7.70-7.78 (m, 2H), 7.99 (d, 1H), 9.78 (s, 1H), 11.44 (br. s, 1H).

### Beispiel 2

### N⁴-{3,5-Difluor-4-[(3-methyl-1H-pyrrolo[2,3-b]pyridin-4-yl)oxy]phenyl}pyrimidin-2,4-diamin

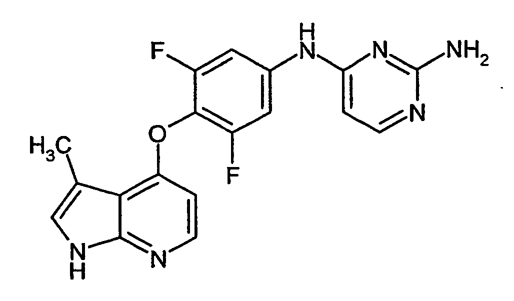

50 mg (0.12 mmol) 6-Chlor-N⁴-{3,5-difluor-4-[(3-methyl-1H-pyrrolo[2,3-b]pyridin-4-yl)oxy]-phenyl}pyrimidin-2,4-diamin werden in 10 ml Ethanol gelöst. Man setzt 35 µl (0.25 mmol) Triethylamin und 26 mg (0.02 mmol) 10%iges Palladium auf Kohle hinzu und hydriert über Nacht unter normalem Druck in einer Wasserstoff-Atmosphäre. Man filtriert dann durch Celite^{®} und wäscht mit Methanol nach. Das Lösungsmittel wird im Vakuum entfernt und das Rohprodukt durch präparative HPLC gereinigt.
Ausbeute: 31 mg (68% d. Th.)
LC-MS (Methode 3): Rₜ = 1.42 min.
MS (ESI pos.): m/z = 369 (M+H)⁺.
¹H-NMR (DMSO-d₆, 300 MHz): δ = 2.44 (s, 3H), 6.02 (d, 1H), 6.20 (d, 1H), 6.42 (br. s, 2H), 7.15 (s, 1H), 7.73-7.80 (m, 2H), 7.89 (d, 1H), 7.99 (d, 1H), 9.53 (s, 1H), 11.39 (br. s, 1H).

### Beispiel 3

### N⁴-{3,5-Difluor-4-[(3-methyl-1H-pyrrolo[2,3-b]pyridin-4-yl)oxy]phenyl}-6-(trifluormethyl)-pyrimidin-2,4-diamin

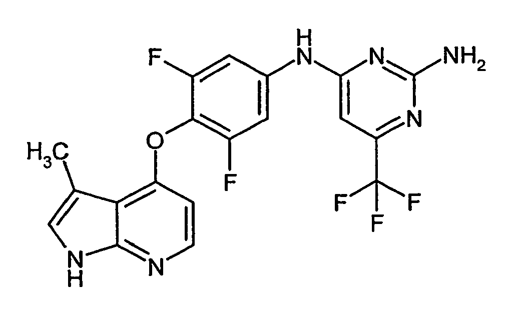

160 mg (0.58 mmol) 3,5-Difluor-4-[(3-methyl-1H-pyrrolo[2,3-b]pyridin-4-yl)oxy]anilin und 138 mg (0.70 mmol) 4-Chlor-6-(trifluormethyl)pyrimidin-2-amin werden in 3.5 ml Wasser suspendiert. Man setzt 0.76 ml (0.76 mmol) 1N Salzsäure hinzu und erhitzt über Nacht auf Rückfluss. Durch Zugabe von 1N Natronlauge stellt man pH10 ein, wobei Kristalle ausfallen. Man saugt ab und reinigt durch Chromatographie an Kieselgel (Eluens: Dichlormethan/Methanol (7N Ammoniak) 100:1 bis 100:3). Das erhaltene Produkt wird durch präparative HPLC weiter aufgereinigt.
Ausbeute: 112 mg (44% d. Th.)
LC-MS (Methode 1): Rₜ = 2.16 min.
MS (ESI pos.): m/z = 437 (M+H)⁺.
¹H-NMR (DMSO-d₆, 400 MHz): δ = 2.44 (s, 3H), 6.22 (d, 1H), 6.37 (s, 1H), 7.11 (br. s, 2H), 7.17 (s, 1H), 7.75-7.82 (m, 2H), 8.00 (d, 1H), 10.03 (s, 1H), 11.44 (br. s, 1H).

### Beispiel 4

### N⁴-{4-[(3-Chlor-1H-pyrrolo[2,3-b]pyridin-4-yl)oxy]-3,5-difluorphenyl}-6-(trifluormethyl)-pyrimidin-2,4-diamin

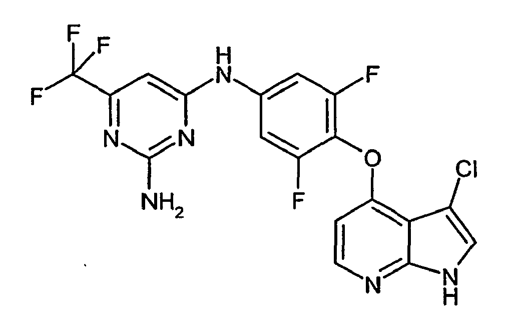

65 mg (0.22 mmol) 3,5-Difluor-4-[(3-chlor-1H-pyrrolo[2,3-b]pyridin-4-yl)oxy]anilin und 47 mg (0.24 mmol) 4-Chlor-6-(trifluormethyl)pyrimidin-2-amin werden in 4 ml Wasser/Ethanol 1:1 suspendiert. Man setzt 25 µl (0.29 mmol) 37%ige Salzsäure hinzu und erhitzt über Nacht auf Rückfluss. Man stellt mit 1N Natronlauge pH7 ein und extrahiert mit Essigsäureethylester. Die organische Phase wird mit gesättigter Natriumchloridlösung gewaschen, über Magnesiumsulfat getrocknet und eingeengt. Man reinigt den Rückstand durch präparative HPLC.
Ausbeute: 40 mg (39% d. Th.)
HPLC (Methode 1): Rₜ = 2.22 min.
MS (ESI pos.): m/z = 439, 441 (M+H)⁺.
¹H-NMR (DMSO-d₆, 300 MHz): δ = 6.28 (d, 1H), 6.38 (s, 1H), 6.99 (br. s, 2H), 7.33 (t, 1H), 7.40 (dd, 1H), 7.58 (s, 1H), 8.10 (d, 1H), 8.25 (dd, 1H), 9.91 (s, 1H), 12.05 (br. s, 1H).

### Beispiel 5

### 6-Chlor-N⁴-{3-fluor-4-[(3-methyl-1H-pyrrolo[2,3-b]pyridin-4-yl)oxy]phenyl}pyrimidin-2,4-diamin

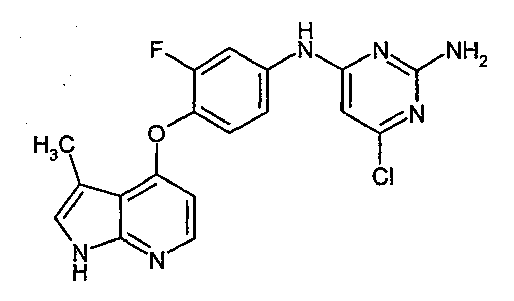

35 mg (0.14 mmol) 3-Fluor-4-[(3-methyl-1H-pyrrolo[2,3-b]pyridin-4-yl)oxy]anilin und 24.5 mg (0.15 mmol) 4,6-Dichlorpyrimidin-2-amin werden in 2.5 ml Wasser suspendiert. Man setzt 17 µl (0.18 mmol) 37%ige Salzsäure hinzu und erhitzt über Nacht auf Rückfluss. Man stellt mit 1N Natronlauge pH10 ein, wobei Kristalle ausfallen. Man saugt ab und reinigt durch Säulenchromatographie an Kieselgel (Eluens: Dichlormethan : Methanol (7N Ammoniak) 100:5).
Ausbeute: 37 mg (67% d. Th.)
LC-MS (Methode 1): Rₜ = 1.73 min.
MS (ESI pos.): m/z = 385 (M+H)⁺.
¹H-NMR (DMSO-d₆, 300 MHz): δ = 2.40 (d, 3H), 6.03 (s, 1H), 6.17 (dd, 1H), 6.84 (br. s, 2H), 7.12 (m, 1H), 7.23-7.36 (m, 2H), 7.98 (d, 1H), 8.14 (dd, 1H), 9.57 (s, 1H), 11.34 (br. s, 1H).

### Beispiel 6

### N⁴-{3-Fluor-4-[(3-methyl-1H-pyrrolo[2,3-b]pyridin-4-yl)oxy]phenyl}pyrimidin-2,4-diamin

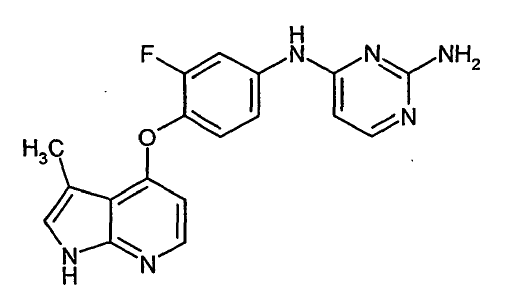

50 mg (0.13 mmol) 6-Chlor-N⁴-{3-fluor-4-[(3-methyl-1H-pyrrolo[2,3-b]pyridin-4-yl)oxy]-phenyl}-pyrimidin-2,4-diamin werden in 10 ml Ethanol gelöst. Man setzt 36 µl (0.26 mmol) Triethylamin und 28 mg (0.03 mmol) 10%iges Palladium auf Kohle hinzu und hydriert über Nacht unter normalem Druck in einer Wasserstoff-Atmosphäre. Man filtriert dann durch Celite^{®} und wäscht mit Methanol nach. Das Lösungsmittel wird im Vakuum entfernt und das Rohprodukt durch präparative HPLC gereinigt.
Ausbeute: 24 mg (53% d. Th.)
LC-MS (Methode 6): Rₜ = 1.48 min.
MS (ESI pos.): m/z = 351 (M+H)⁺.
¹H-NMR (DMSO-d₆, 300 MHz): δ = 2.41 (d, 3H), 6.02 (d, 1H), 6.16 (dd, 1H), 6.30 (br. s, 2H), 7.12 (s, 1H), 7.21-7.40 (m, 2H), 7.85 (d, 1H), 7.97 (d, 1H), 8.18 (dd, 1H), 9.36 (s, 1H), 11.33 (br. s, 1H).

### Beispiel 7

### N⁴-{3-Fluor-4-[(3-methyl-1H-pyrrolo[2,3-b]pyridin-4-yl)oxy]phenyl}-6-(trifluormethyl)pyrimidin-2,4-diamin

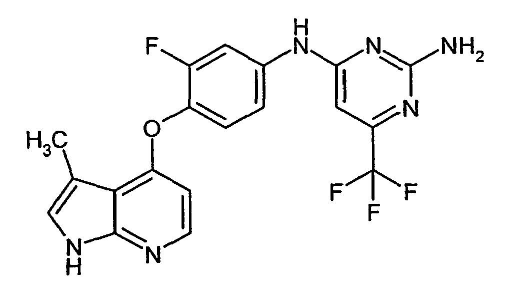

184 mg (0.72 mmol) 3-Fluor-4-[(3-methyl-1H-pyrrolo[2,3-b]pyridin-4-yl)oxy]anilin und 170 mg (0.86 mmol) 4-Chlor-6-(trifluormethyl)pyrimidin-2-amin werden in 3.5 ml Wasser suspendiert. Man setzt 0.93 ml (0.93 mmol) 1N Salzsäure hinzu und erhitzt über Nacht auf Rückfluss. Die Suspension wird mit 1N Natronlauge auf pH10 gestellt, wobei Kristalle ausfallen. Man saugt ab, wäscht mit Wasser nach und reinigt durch Säulenchromatographie an Kieselgel (Eluens: Dichlormethan/Methanol (7N Ammoniak) 100:1 bis 100:3).
Ausbeute: 212 mg (71% d. Th.)
LC-MS (Methode 1): Rₜ= 1.97 min.
MS (ESI pos.): m/z = 419 (M+H)⁺.
¹H-NMR (DMSO-d₆, 400 MHz): δ = 2.41 (s, 3H), 6.17 (d, 1H), 6.38 (s, 1H), 7.00 (br. s, 2H), 7.14 (s, 1H), 7.30 (t, 1H), 7.38 (dd, 1H), 7.98 (d, 1H), 8.23 (dd, 1H), 9.87 (s, 1H), 11.38 (br. s, 1H).

### Beispiel 8

### 6-Chlor-N⁴-{4-[(3-chlor-1H-pyrrolo[2,3-b]pyridin-4-yl)oxy]-3-fluorphenyl}pyrimidin-2,4-diamin

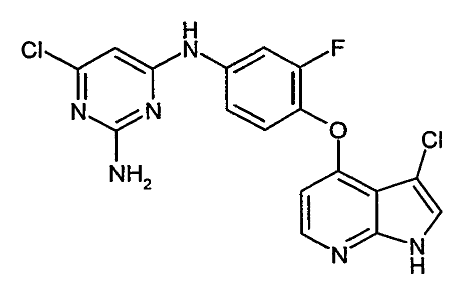

67 mg (0.24 mmol) 3-Fluor-4-[(3-chlor-1H-pyrrolo[2,3-b]pyridin-4-yl)oxy]anilin und 40 mg (0.24 mmol) 4,6-Dichlorpyrimidin-2-amin werden in 4 ml Wasser/Ethanol 1:1 suspendiert. Man setzt 25 µl (0.29 mmol) 37%ige Salzsäure hinzu und erhitzt über Nacht auf Rückfluss. Man stellt mit 1N Natronlauge pH7 ein und extrahiert mit Essigsäureethylester. Die organische Phase wird mit gesättigter Natriumchloridlösung gewaschen, mit Magnesiumsulfat getrocknet und eingeengt. Man reinigt den Rückstand durch präparative HPLC.
Ausbeute: 54 mg (53% d. Th.)
HPLC (Methode 1): Rₜ = 2.07 min.
MS (ESI pos.): m/z = 405, 407 (M+H)⁺.
¹H-NMR (DMSO-d₆, 300 MHz): δ = 6.05 (s, 1H), 6.27 (dd, 1H), 6.84 (br. s, 2H), 7.29 (t, 1H), 7.36 (m, 1H), 7.56 (s, 1H), 8.09 (d, 1H), 8.16 (dd, 1H), 9.62 (s, 1H), 12.03 (br. s, 1H).

### Beispiel 9

### N⁴-{4-[(3-Chlor-1H-pyrrolo[2,3-b]pyridin-4-yl)oxy]-3-fluorphenyl}-6-(trifluormethyl)pyrimidin-2,4-diamin

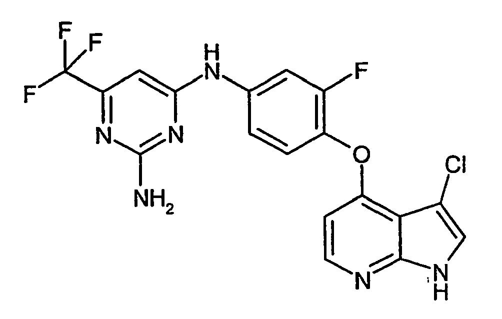

67 mg (0.24 mmol) 3-Fluor-4-[(3-chlor-1H-pyrrolo[2,3-b]pyridin-4-yl)oxy]anilin und 47 mg (0.24 mmol) 4-Chlor-6-(trifluormethyl)pyrimidin-2-amin werden in 4 ml Wasser/Ethanol 1:1 suspendiert. Man setzt 25 µl (0.29 mmol) 37%ige Salzsäure hinzu und erhitzt über Nacht auf Rückfluss. Man stellt mit 1N Natronlauge pH7 ein und extrahiert mit Essigsäureethylester. Die organische Phase wird mit gesättigter Natriumchloridlösung gewaschen, mit Magnesiumsulfat getrocknet und eingeengt. Man reinigt den Rückstand durch präparative HPLC.
Ausbeute: 40 mg (36% d. Th.)
HPLC (Methode 1): Rₜ = 2.22 min.
MS (ESI pos.): m/z = 439, 441 (M+H)⁺.
¹H-NMR (DMSO-d₆, 300 MHz): δ = 6.28 (d, 1H), 6.38 (s, 1H), 6.99 (br. s, 2H), 7.33 (t, 1H), 7.40 (dd, 1H), 7.58 (s, 1H), 8.10 (d, 1H), 8.25 (dd, 1H), 9.91 (s, 1H), 12.05 (br. s, 1H).

### Beispiel 10

### N⁴-{4-[(3-Chlor-1H-pyrrolo[2,3-b]pyridin-4-yl)oxy]-3-fluorphenyl}pyrimidin-2,4-diamin

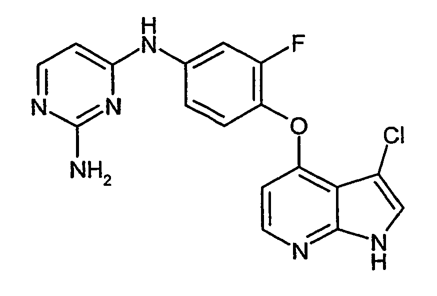

100 mg (0.36 mmol) 4-[(3-Chlor-1H-pyrrolo[2,3-b]pyridin-4-yl)oxy]-3-fluoranilin und 60 mg (0.46 mmol) 4-Chlorpyrimidin-2-amin werden in 4 ml Wasser/Ethanol 1:1 suspendiert. Man setzt 36 µl (0.43 mmol) 37%ige Salzsäure hinzu und erhitzt über Nacht auf Rückfluss. Man stellt mit 1N Natronlauge pH7 ein, und extrahiert mit Essigsäureethylester. Die organische Phase wird mit gesättigter Natriumchloridlösung gewaschen, über Magnesiumsulfat getrocknet und eingeengt. Man reinigt den Rückstand durch präparative HPLC.
Ausbeute: 68 mg (51 % d. Th.)
HPLC (Methode 1): Rₜ =1.42 min.
MS (ESI pos.): m/z = 371, 373 (M+H)⁺.
¹H-NMR (DMSO-d₆, 300 MHz): δ = 6.05 (d, 1H), 6.26 (dd, 1H), 6.83 (br. s, 2H), 7.28 (t, 1H), 7.37 (m, 1H), 7.56 (s, 1H), 7.89 (d, 1H), 8.09 (d, 1H), 8.15 (dd, 1H), 9.12 (s, 1H), 12.07 (br. s, 1H).

### Beispiel 11

### N⁴-{4-[(3-Cyclopropyl-1H-pyrrolo[2,3-b]pyridin-4-yl)oxy]-3-fluorphenyl}-6-(trifluormethyl)-pyrimidin-2,4-diamin

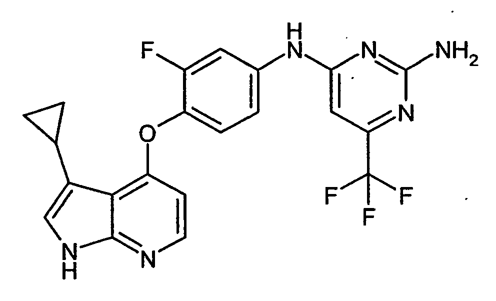

114 mg (0.40 mmol) 4-[(3-Cyclopropyl-1H-pyrrolo[2,3-b]pyridin-4-yl)oxy]-3-fluoranilin und 95 mg (0.48 mmol) 4-Chlor-6-(trifluormethyl)pyrimidin-2-amin werden in 3.5 ml Wasser suspendiert. Man setzt 0.52 ml (0.52 mmol) 1N Salzsäure hinzu und erhitzt über Nacht auf Rückfluss. Danach gibt man erneut 30 mg (0.15 mmol) 4-Chlor-6-(trifluormethyl)pyrimidin-2-amin zu und erhitzt weitere 6 Stunden. Durch Zugabe von 1N Natronlauge stellt man pH10 ein, wobei Kristalle ausfallen. Man saugt ab und reinigt durch Chromatographie an Kieselgel (Eluens: Dichlormethan/Methanol (7N Ammoniak) 100:1 bis 100:3).
Ausbeute: 94 mg (53% d. Th.)
LC-MS (Methode 6): Rₜ = 2.31 min.
MS (ESI pos.): m/z = 445 (M+H)⁺.
¹H-NMR (DMSO-d₆, 300 MHz): δ = 0.59-0.65 (m, 2H), 0.77-0.85 (m, 2H), 2.10-2.20 (m, 1H), 6.21 (dd, 1H), 6.38 (s, 1H), 6.95 (br. s, 2H), 7.04 (d, 1H), 7.25-7.41 (m, 2H), 7.99 (d, 1H), 8.21 (dd, 1H), 9.83 (s, 1H), 11.38 (br. s, 1H).

### Beispiel 12

### 6-Chlor-N⁴-{4-[(3-ethyl-1H-pyrrolo[2,3-b]pyridin-4-yl)oxy]-3-fluorphenyl}pyrimidin-2,4-diamin

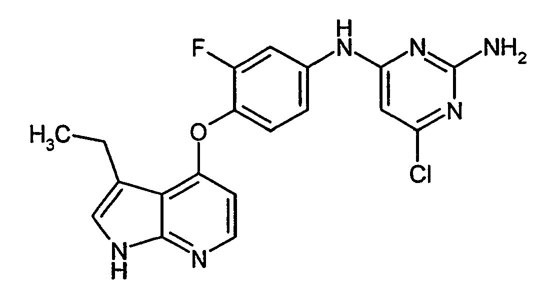

50 mg (0.18 mmol) 4-[(3-Ethyl-1H-pyrrolo[2,3-b]pyridin-4-yl)oxy]-3-fluoranilin und 32 mg (0.19 mmol) 4,6-Dichlorpyrimidin-2-amin werden in 5 ml Wasser suspendiert. Man setzt 27 µl 37%ige Salzsäure hinzu und erhitzt über Nacht auf Rückfluss. Dann wird mit 1N Natronlauge pH10 eingestellt, wobei Kristalle ausfallen. Man saugt ab und reinigt durch präparative HPLC.
Ausbeute: 26 mg (33% d. Th.)
LC-MS (Methode 1): Rₜ = 1.93 min.
MS (ESI pos.): m/z = 399 (M+H)⁺.
¹H-NMR (DMSO-d₆, 300 MHz): δ = 1.27 (t, 3H), 2.83 (q, 2H), 6.04 (s, 1H), 6.16 (d, 1H), 6.83 (br. s, 2H), 7.14 (m, 1H), 7.24-7.37 (m, 2H), 7.98 (d, 1H), 8.14 (dd, 1H), 9.57 (s, 1H), 11.36 (br. s, 1H).

### Beispiel 13

### N⁴-{4-[(3-Ethyl-1H-pyrrolo[2,3-b]pyridin-4-yl)oxy]-3-fluorphenyl}-6-(trifluormethyl)pyrimidin-2,4-diamin

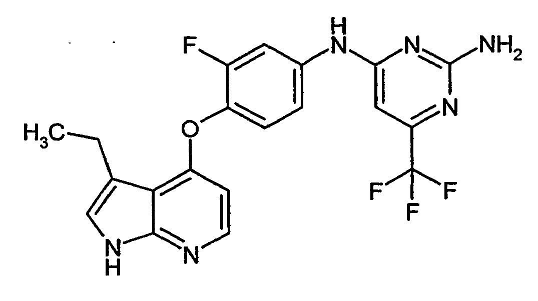

45 mg (0.17 mmol) 4-[(3-Ethyl-1H-pyrrolo[2,3-b]pyridin-4-yl)oxy]-3-fluoranilin und 34 mg (0.17 mmol) 4-Chlor-6-(trifluormethyl)pyrimidin-2-amin werden in 5 ml Wasser suspendiert. Man setzt 24 µl 37%ige Salzsäure hinzu und erhitzt über Nacht auf Rückfluss. Danach werden nochmals 70 mg (0.35 mmol) 4-Chlor-6-(trifluormethyl)pyrimidin-2-amin und 40 µl 37%ige Salzsäure zugesetzt, und es wird eine weitere Nacht auf Rückfluss erhitzt. Dann wird mit 1N Natronlauge pH10 eingestellt. Man engt im Vakuum ein und reinigt den Rückstand durch Säulenchromatographie an Kieselgel (Eluens: Dichlormethan/Methanol 100:1 bis 10: 1). Das erhaltene Produkt wird durch präparative HPLC weiter aufgereinigt.
Ausbeute: 9.6 mg (13% d. Th.)
LC-MS (Methode 3): Rₜ = 2.12 min.
MS (ESI pos.): m/z = 433 (M+H)⁺.
¹H-NMR (DMSO-d₆, 400 MHz): δ = 1.28 (t, 3H), 2.83 (q, 2H), 6.17 (d, 1H), 6.38 (s, 1H), 7.00 (br. s, 2H), 7.15 (br. s, 1H), 7.28-7.40 (m, 2H), 7.99 (d, 1H), 8.23 (dd, 1H), 9.88 (s, 1H), 11.40 (br. s, 1H).

### Beispiel 14

### 2-(4-{4-[(2-Amino-6-chlorpyrimidin-4-yl)amino]-2-fluorphenoxy}-1H-pyrrolo[2,3-b]pyridin-3-yl)ethanol

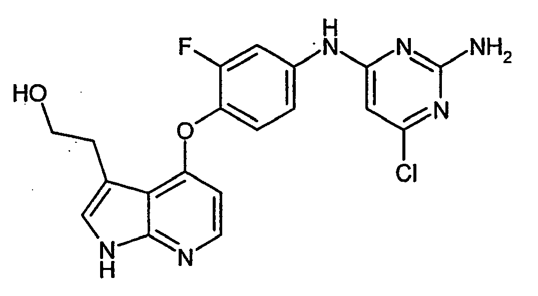

50 mg (0.17 mmol) 2-[4-(4-Amino-2-fluorphenoxy)-1H-pyrrolo[2,3-b]pyridin-3-yl]ethanol und 30 mg (0.18 mmol) 4,6-Dichlorpyrimidin-2-amin werden in 5 ml Wasser suspendiert. Man setzt 25 µl 37% ige Salzsäure hinzu und erhitzt über Nacht auf Rückfluss. Dann wird mit 1N Natronlauge pH10 eingestellt, wobei Kristalle ausfallen. Man engt ein und filtriert durch Kieselgel. Man reinigt durch präparative HPLC.
Ausbeute: 16 mg (22% d. Th.)
LC-MS (Methode 3): Rₜ = 1.52 min.
MS (ESI pos.): m/z = 415 (M+H)⁺,
¹H-NMR (DMSO-d₆, 300 MHz): δ = 2.97 (t, 2H), 3.70 (dt, 2H), 4.52 (t, 1H), 6.04 (s, 1H), 6.15 (d, 1H), 6.84 (br. s, 2H), 7.17 (d, 1H), 7.25-7.37 (m, 2H), 7.98 (d, 1H), 8.15 (dd, 1H), 9.58 (s, 1H), 11.41 (br. s, 1H).

### Beispiel 15

### 2-[4-(4-{[2-Amino-6-(trifluormethyl)pyrimidin-4-yl]amino}-2-fluorphenoxy)-1H-pyrrolo[2,3-b]pyridin-3-yl]ethanol

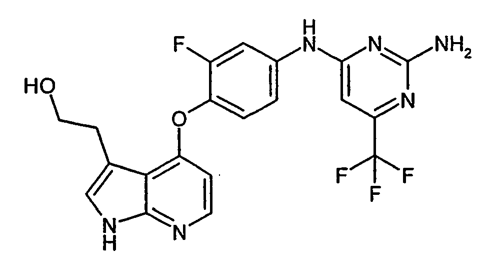

130 mg (0.45 mmol) 2-[4-(4-Amino-2-fluorphenoxy)-1H-pyrrolo[2,3-b]pyridin-3-yl]ethanol und 107 mg (0.54 mmol) 4-Chlor-6-(trifluormethyl)pyrimidin-2-amin werden in 6 ml Wasser suspendiert. Man setzt 0.6 ml (0.6 mmol) 1N Salzsäure hinzu und erhitzt über Nacht auf Rückfluss. Die Suspension wird mit 1N Natronlauge auf pH10 gestellt, wobei Kristalle ausfallen. Man saugt ab, wäscht mit Wasser nach und reinigt durch Säulenchromatographie an Kieselgel (Eluens: Dichlormethan/Methanol (7N Ammoniak) 100:1 bis 100:3).
Ausbeute: 170 mg (80% d. Th.)
LC-MS (Methode 1): Rₜ= 1.54 min.
MS (ESI pos.): m/z = 449 (M+H)⁺.
¹H-NMR (DMSO-d₆, 400 MHz): δ = 2.97 (t, 2H), 3.70 (dt, 2H), 4.58 (t, 1H), 6.16 (d, 1H), 6.38 (s, 1H), 7.10 (br. s, 2H), 7.18 (d, 1H), 7.29-7.41 (m, 2H), 7.98 (d, 1H), 8.24 (dd, 1H), 9.89 (s, 1H), 11.46 (br. s, 1H).

### Beispiel 16

### 2-(4-{4-[(2-Aminopyrimidin-4-yl)amino]-2-fluorphenoay}-1H-pyrrolo[2,3-b]pyridin-3-yl)ethanol

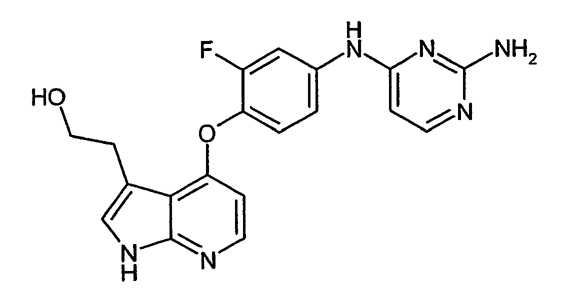

135 mg (0.47 mmol) 2-[4-(4-Amino-2-fluorphenoxy)-1H-pyrrolo[2,3-b]pyridin-3-yl]ethanol und 79 mg (0.61 mmol) 4-Chlorpyrimidin-2-amin werden in 6 ml Wasser suspendiert. Man setzt 0.6 ml (0.6 mmol) 1N Salzsäure hinzu und erhitzt über Nacht auf Rückfluss. Die Suspension wird mit 1N Natronlauge auf pH10 gestellt, wobei Kristalle ausfallen. Man saugt ab, wäscht mit Wasser nach und reinigt durch Säulenchromatographie an Kieselgel (Eluens: Dichlormethan/Methanol (7N Ammoniak) 100:1 bis 10:1).
Ausbeute: 158 mg (88% d. Th.)
LC-MS (Methode 1): Rₜ = 0.88 min.
MS (ESI neg.): m/z = 379 (M-H)⁻.
¹H-NMR (DMSO-d₆, 400 MHz): δ = 2.97 (t, 2H), 3.70 (dt, 2H), 4.58 (t, 1H), 6.02 (d, 1H), 6.14 (d, 1H), 6.36 (br. s, 2H), 7.17 (d, 1H), 7.24-7.40 (m, 2H), 7.86 (d, 1H), 7.98 (d, 1H), 8.22 (dd, 1H), 9.41 (s,1H),11.44(br.s,1H).

### Beispiel 17

### N⁴-(3-Fluor-4-{[3-(2-methoxyethyl)-1H-pyrrolo[2,3-b]pyridin-4-yl]oxy}phenyl)-6-(trifluor-methyl)pyrimidin-2,4-diamin

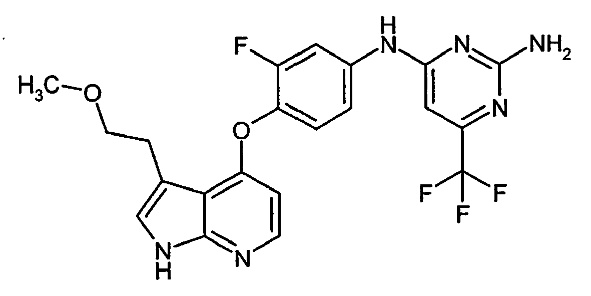

53 mg (0.18 mmol) 3-Fluor-4-{[3-(2-methoxyethyl)-1H-pyrrolo[2,3-b]pyridin-4-yl]oxy}anilin und 42 mg (0.21 mmol) 4-Chlor-6-(trifluormethyl)pyrimidin-2-amin werden in 4 ml Wasser suspendiert. Man setzt 0.23 ml (0.23 mmol) 1N Salzsäure hinzu und erhitzt über Nacht auf Rückfluss. Dann werden weitere 22 mg (0.11 mmol) 4-Chlor-6-(trifluormethyl)pyrimidin-2-amin zugesetzt und nochmals über Nacht erhitzt. Durch Zugabe von 1N Natronlauge stellt man pH10 ein, wobei Kristalle ausfallen. Man saugt ab und wäscht mit Wasser nach. Man reinigt durch präparative HPLC.
Ausbeute: 45 mg (55% d. Th.)
LC-MS (Methode 1): Rₜ =1.88 min.
MS (ESI pos.): m/z = 463 (M+H)⁺.
¹H-NMR (DMSO-d₆, 300 MHz): δ = 3.04 (t, 2H), 3.25 (s, 3H), 3.64 (t, 2H), 6.19 (d, 1H), 6.38 (s, 1H), 6.96 (br. s, 2H), 7.20 (d, 1H), 7.28-7.42 (m, 2H), 8.00 (d, 1H), 8.22 (dd, 1H), 9.85 (s, 1H), 11.46 (br. s, 1H).

### Beispiel 18

### 4-(4-{[2-Amino-6-(trifluormethyl)pyrimidin-4-yl]amino}-2-fluorphenoay)-1H-pyrrolo[2,3-b]pyridin-3-carbonitril

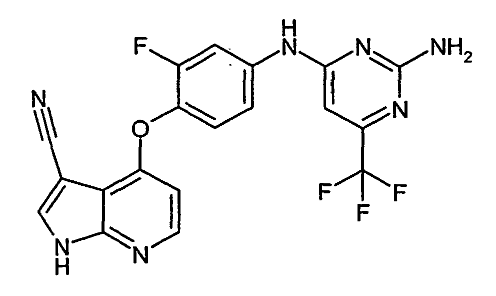

19.0 mg (0.071 mmol) 4-(4-Amino-2-fluorphenoxy)-1H-pyrrolo[2,3-b]pyridin-3-carbonitril und 19.6 mg (0.099 mmol) 4-Chlor-6-(trifluormethyl)pyrimidin-2-amin werden in 5 ml Wasser suspendiert. Man setzt 0.1 ml (0.1 mmol) 1N Salzsäure hinzu und erhitzt über Nacht auf Rückfluss. Danach werden 3 ml Ethanol und weitere 19.6 mg (0.099 mmol) 4-Chlor-6-(trifluormethyl)pyrimidin-2-amin zugesetzt. Man lässt weitere 6 Stunden bei 100°C rühren. Man engt etwas ein und stellt mit 1N Natronlauge auf pH10, wobei Kristalle ausfallen. Man saugt ab, wäscht mit Wasser nach und reinigt durch Säulenchromatographie an Kieselgel (Eluens: Dichlormethan/Methanol (7N Ammoniak) 100:1 bis 10:1).
Ausbeute: 24 mg (77% d. Th.)
LC-MS (Methode 1): Rₜ = 1.93 min.
MS (ESI pos.): m/z = 430 (M+H)⁺.
¹H-NMR (DMSO-d₆, 300 MHz): δ = 6.39 (s, 1H), 6.44 (d, 1H), 7.04 (br. s, 2H), 7.40-7.43 (m, 2H), 8.22 (d, 1H), 8.29 (d, 1H), 8.43 (s, 1H), 9.93 (s, 1H), 12.93 (br. s, 1H).

### Beispiel 19

### 4-(4-{[2-Amino-6-chlorpyrimidin-4-yl]amino}-2-fluorphenoxy)-1H-pyrrolo[2,3-b]pyridin-3-carbonitril

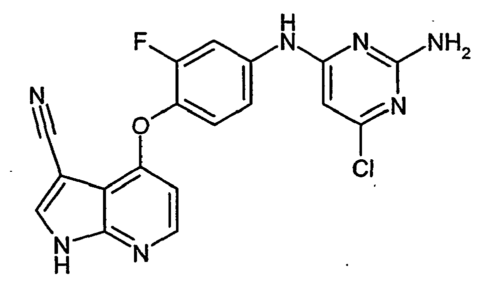

49.0 mg (0.18 mmol) 4-(4-Amino-2-fluorphenoxy)-1H-pyrrolo[2,3-b]pyridin-3-carbonitril und 33.0 mg (0.20 mmol) 4,6-Dichlorpyrimidin-2-amin werden in 5 ml Wasser suspendiert. Man setzt 0.12 ml (0.24 mmol) 2N Salzsäure hinzu und erhitzt über Nacht auf Rückfluss. Man stellt mit konzentrierter Natronlauge alkalisch und reinigt durch präparative HPLC.
Ausbeute: 40 mg (54% d. Th.)
LC-MS (Methode 3): Rₜ = 2.05 min.
MS (ESI pos.): m/z = 396 (M+H)⁺.
¹H-NMR (DMSO-d₆, 400 MHz): δ = 6.05 (s, 1H), 6.41 (d, 1H), 6.89 (br. s, 2H), 7.37 (m, 2H), 8.18-8.24 (m, 2H), 8.38 (s, 1H), 9.68 (s, 1H), 13.00 (br. s, 1H).

### Beispiel 20

### 4-{4-[(2-Aminopyrimidin-4-yl)amino]-2-fluorphenoxy}-1H-pyrrolo[2,3-b]pyridin-3-carbonitril

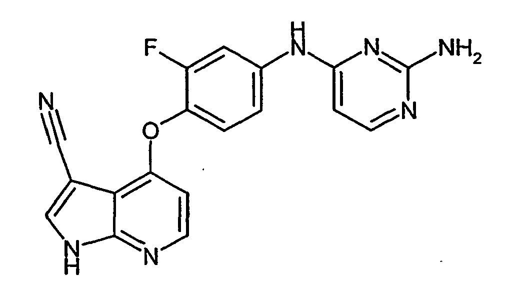

200 mg (0.66 mmol) 4-(4-Amino-2-fluorphenoxy)-1H-pyrrolo[2,3-b]pyridin-3-carbonitril und 104 mg (0.72 mmol) 4-Chlorpyrimidin-2-amin werden in 9 ml Wasser und 4.5 ml Ethanol suspendiert. Man setzt 0.33 ml (1.31 mmol) 4N Salzsäure hinzu und erhitzt 2 h auf Rückfluss. Man stellt mit konzentrierter Natronlauge alkalisch, verdünnt mit Wasser und extrahiert mit Essigsäureethylester. Die organische Phase wird abgetrennt und eingeengt. Das Rohprodukt wird durch präparative HPLC gereinigt. Man erhält die Titelverbindung als Kristalle.
Ausbeute: 81 mg (34% d. Th.)
LC-MS (Methode 1): Rₜ=1.24 min.
MS (ESI pos.): m/z = 362 (M+H)⁺.
¹H-NMR (DMSO-d₆, 400 MHz): δ = 6.03 (d, 1H), 6.36 (br. s, 2H), 6.42 (d, 1H), 7.32-7.43 (m, 2H), 7.86 (d, 1H), 8.21 (d, 1), 8.26 (dd, 1H), 8.41 (s, 1H), 9.44 (s, 1H), 12.90 (s, 1H).

### Beispiel 21

### 4-{4-[(2-Aminopyrimidin-4-yl)amino]-2,6-difluorphenoxy}-1H-pyrrolo[2,3-b]pyridin-3-carbonitril

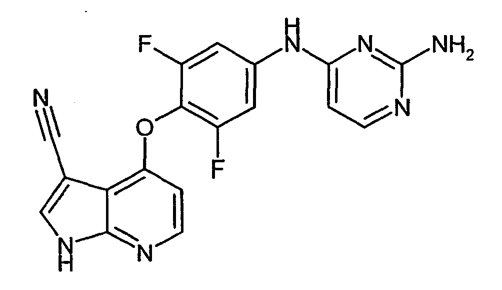

250 mg (0.65 mmol) 4-(4-Amino-2,6-difluorphenoxy)-1H-pyrrolo[2,3-b]pyridin-3-carbonitril und 92 mg (0.71 mmol) 4-Chlorpyrimidin-2-amin werden in 10 ml Wasser und 5 ml Ethanol suspendiert. Man setzt 0.32 ml (1.29 mmol) 4N Salzsäure hinzu und erhitzt 2 h auf Rückfluss. Man stellt mit konzentrierter Natronlauge alkalisch, verdünnt mit Wasser und extrahiert mit Essigsäureethylester. Die organische Phase wird abgetrennt und eingeengt. Das Rohprodukt wird durch präparative HPLC gereinigt. Man erhält die Titelverbindung als Kristalle.
Ausbeute: 138 mg (56% d. Th.)
LC-MS (Methode 2): Rₜ = 1.48 min.
MS (ESI pos.): m/z = 380 (M+H)⁺.
¹H-NMR (DMSO-d₆, 400 MHz): δ = 6.03 (d, 1H), 6.49 (br. s, 2H), 6.54 (d, 1R), 7.82 (d, 2H), 7.91 (d, 1H), 8.24 (d, 1H), 8.45 (s, 1H), 9.61 (s, 1H), 12.98 (br. s, 1H).

### Beispiel 22

### N⁴-{4-[(3-Cyclopropyl-1H-pyrrolo[2,3-b]pyridin-4-yl)oxy]-3,5-difluorphenyl}-6-(trifluormethyl)-pyrimidin-2,4-diamin

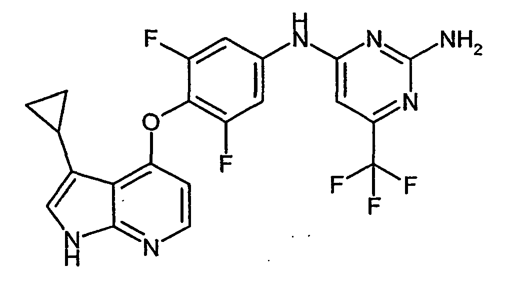

330 mg (1.10 mmol) 4-[(3-Cyclopropyl-1H-pyrrolo[2,3-b]pyridin-4-yl)oxy]-3,5-difluoranilin und 320 mg (1.64 mmol) 4-Chlor-6-(trifluormethyl)pyrimidin-2-amin werden in 4 ml Wasser und 2 ml Ethanol suspendiert. Man setzt 0.55 ml (2.2 mmol) 4N Salzsäure hinzu und erhitzt 1 h auf Rückfluss. Danach gibt man erneut 107 mg (0.55 mmol) 4-Chlor-6-(trifluormethyl)pyrimidin-2-amin zu und erhitzt weitere 2 Stunden. Durch Zugabe von 1N Natronlauge stellt man alkalisch, extrahiert mit Essigsäureethylester und wäscht die organische Phase mit Wasser. Man trocknet über Natriumsulfat und entfernt das Lösungsmittel im Vakuum. Man reinigt durch präparative HPLC.
Ausbeute: 340 mg (65% d. Th.)
LC-MS (Methode 2): Rₜ = 2.45 min.
MS (ESI pos.): m/z = 463 (M+H)⁺.
¹H-NMR (DMSO-d₆, 300 MHz): δ = 0.61-0.68 (m, 2H), 0.81-0.89 (m, 2H), 2.14-2.25 (m, 1H), 6.25 (d, 1H), 6.38 (s, 1H), 7.09 (d, 1H), 7.12 (br. s, 2H), 7.79 (d, 2H), 8.01 (d, 1H), 10.05 (br. s, 1H), 11.48 (br. s, 1H).

### Beispiel 23

### 6-Chlor-N⁴-{4-[(3-chlor-1H-pyrrolo[2,3-b]pyridin-4-yl)oxy]-3,5-difluorphenyl}pyrimidin-2,4-diamin

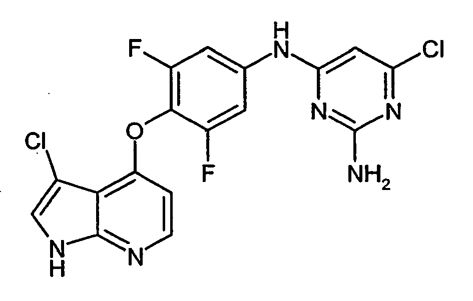

162 mg (0.55 mmol) 3,5-Difluor-4-[(3-chlor-1H-pyrrolo[2,3-b]pyridin-4-yl)oxy]anilin und 90 mg (0.55 mmol) 4,6-Dichlorpyrimidin-2-amin werden in 6 ml Wasser/Ethanol 1:1 suspendiert. Man setzt 56 µl (0.65 mmol) 37%ige Salzsäure hinzu und erhitzt 3 h auf Rückfluss. Man stellt mit 1N Natronlauge pH7 ein und extrahiert mit Essigsäureethylester. Die organische Phase wird mit gesättigter Natriumchloridlösung gewaschen, über Magnesiumsulfat getrocknet und eingeengt. Man reinigt den Rückstand durch präparative HPLC.
Ausbeute: 79 mg (34% d. Th.)
HPLC (Methode 3): Rₜ = 2.38 min.
MS (ESI pos.): m/z = 405, 407 (M+H)⁺.
¹H-NMR (DMSO-d₆, 300 MHz): δ = 6.04 (s, 1H), 6.35 (d, 1H), 7.00 (br. s, 2H), 7.61 (s, 1H), 7.75 (d, 2H), 8.10 (d, 1H), 9.80 (s, 1H), 12.13 (br. s, 1H).

### Beispiel 24

### N⁴-{4-[(3-Chlor-1H-pyrrolo[2,3-b]pyridin-4-yl)oxy]-3,5-difluorphenyl}pyrimidin-2,4-diamin

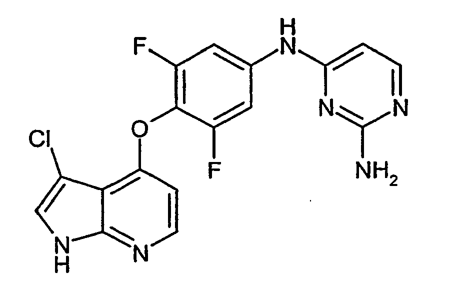

200 mg (0.67 mmol) 3,5-Difluor-4-[(3-chlor-1H-pyrrolo[2,3-b]pyridin-4-yl)oxy]anilin und 88 mg (0.67 mmol) 4-Chlorpyrimidin-2-amin werden in 6 ml Wasser/Ethanol 1:1 suspendiert. Man setzt 69 µl (0.81 mmol) 37%ige Salzsäure hinzu und erhitzt 3 h auf Rückfluss. Man stellt mit 1N Natronlauge pH7 ein und extrahiert mit Essigsäureethylester. Die organische Phase wird mit gesättigter Natriumchloridlösung gewaschen, über Magnesiumsulfat getrocknet und eingeengt. Man reinigt den Rückstand durch präparative HPLC.
Ausbeute: 93 mg (35% d. Th.)
HPLC (Methode 2): Rₜ = 1.63 min.
MS (ESI pos.): m/z = 405, 407 (M+H)⁺.
¹H-NMR (DMSO-d₆, 300 MHz): δ = 6.02 (d, 1H), 6.35 (d, 1H), 6.47 (br. s, 2H), 7.61 (s, 1H), 7.79 (d, 2H), 7.90 (d, 1H), 8.10 (d, 1H), 9.58 (s, 1H), 12.12 (br. s, 1H).

### Beispiel 25

### N⁴-{4-[(3-Brom-1H-pyrrolo[2,3-b]pyridin-4-yl)oxy]-3,5-difluorphenyl}-6-(trifluormethyl)pyrimidin-2,4-diamin

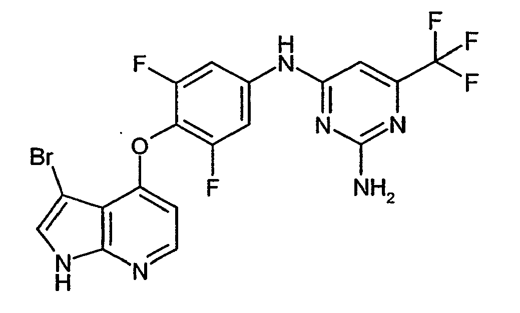

47 mg (0.14 mmol) 4-[(3-Brom-1H-pyrrolo[2,3-b]pyridin-4-yl)oxy]-3,5-difluoranilin und 35 mg (0.18 mmol) 4-Chlor-6-(trifluormethyl)pyrimidin-2-amin werden in einer Mischung aus 5 ml Wasser und 1 ml Ethanol suspendiert. Man setzt 0.18 ml 1M Salzsäure hinzu, und es wird über Nacht auf 100°C erhitzt. Die Suspension wird dann mit 1N Natronlauge auf pH 10 gestellt, wobei Kristalle ausfallen. Der Feststoff wird abfiltriert und mit Wasser gewaschen. Das Rohprodukt wird durch präparative HPLC gereinigt.
Ausbeute: 18 mg (26% d. Th.)
LC-MS (Methode 2): Rₜ = 2.50 min.
MS (ESI neg.): m/z = 499 (M-H)⁻.
¹H-NMR (CD₃OD/CD₂Cl₂ 1:1, 400 MHz): δ = 5.48 (s, 2H), 6.36 (d, 1H), 6.42 (s, 1H), 7.37 (s, 1H), 7.68 (d, 2H), 8.06 (d, 1H).

### B. Bewertung der physiologischen Wirksamkeit

In einem in vitro-Assay mit rekombinanter Rho-Kinase-II wird die Hemmung des Enzyms untersucht. Die gefäßrelaxierende Wirkung wird an Phenylephrin-induzierten Kontraktionen isolierter Ringe der Kaninchen-Arteria-Saphena bestimmt. Die Eignung der erfindungsgemäßen Verbindungen zur Behandlung von kardiovaskulären Erkrankungen kann durch Untersuchung der blutdrucksenkenden Wirkung an narkotisierten Ratten gezeigt werden.

### Hemmung der rekombinanten Rho-Kinase II (ROKα)

Die Aktivität der Rho-Kinase wird durch den Einbau von ³³P-Phosphat in ein Substratpeptid bestimmt. Dazu wird kommerziell erhältliche Rho-Kinase II (Upstate Biotechnology) in Gegenwart des S6 Phosphate-Acceptor-Peptides mit den Testsubstanzen oder einer Lösungsmittelkontrolle 10 min bei 37°C vorinkubiert. Anschließend wird die Kinase-Reaktion durch Zugabe von ³³P-markiertem ATP gestartet. Nach 20 min bei 37°C wird die Reaktion durch Zugabe von H₃PO₄ gestoppt. Aliquots werden auf Filter pipettiert, die Filter gewaschen und anschließend mit Scintillator beschichtet. Die Radioaktivität der am Filter gebundenen ³³P-markierten Peptide wird in einem Micro-Beta-Counter gemessen. Der IC₅₀-Wert entspricht der Konzentration einer Testsubstanz bei welcher der Rho-Kinase katalysierte Einbau von ³³P in das Peptid im Vergleich zu einer Lösungsmittelkontrolle um 50% gehemmt ist. Die experimentellen Daten sind in der folgenden Tabelle A zusammengestellt.

**Tabelle A:**

| **Beispiel-Nr.** | **IC₅₀ (nM)** |
|---|---|
| **1** | 2 |
| **5** | 7 |
| **9** | 4 |
| **18** | 2 |

### Gefäßrelaxierende Wirkung in vitro

3 mm breite Ringe der isolierten Kaninchen-Arteria-Saphena werden einzeln in 5 ml-Organbäder mit 37°C warmer, carbogenbegaster Krebs-Henseleit-Lösung gebracht. Der Gefäßtonus wird isometrisch erfasst und registriert. Kontraktionen werden durch Zugabe von 3x10⁻⁸ g/ml Phenylephrin induziert und nach 4 min wieder ausgewaschen. Nach mehreren Kontrollzyklen wird die zu untersuchende Substanz mit jedem weiteren Durchgang in steigender Dosierung vorinkubiert und die darauffolgende Kontraktion mit der Höhe der letzten Kontrollkontraktion verglichen. Es wird die Konzentration errechnet, die erforderlich ist, um die Höhe des Kontrollwertes um 50% zu reduzieren (IC₅ₒ). Die experimentellen Daten sind in der folgenden Tabelle B zusammengestellt.

**Tabelle B:**

| **Beispiel-Nr.** | **IC₅₀ (nM)** |
|---|---|
| **1** | 67 |
| **5** | 100 |
| **9** | 45 |
| **18** | 19 |

### Blutdruckmessung an narkotisierten Ratten

Männliche Wistar-Ratten mit einem Körpergewicht von 300 - 350 g werden mit Thiopental (100 mg/kg i.p.) anästhesiert. Nach der Tracheotomie wird in die Femoralarterie ein Katheter zur Blutdruckmessung eingeführt. Die zu prüfenden Substanzen werden als Lösungen entweder oral mittels Schlundsonde oder über die Femoralvene intravenös verabreicht.

### Inhibition von Cytochrom P-450 Enzymen (CYP Assay)

Das Potential der Inhibition von, den für den Metabolismus wichtigen, P-450 Isoenzymen (1A2, 2C9, 2D6 und 3A4) wird automatisiert im 96-well Format untersucht. Es werden humane Lebermikrosomen als Enzymquelle und die CYP Isoform-selektiven Substrate Phenacetin (CYP1A2), Diclofenac (CYP2C9), Dextromethorphan (CYP2D6) und Midazolam (CYP3A4) verwendet. Die Bildung des jeweiligen Metaboliten aus den jeweiligen Standard-Substraten wird mittels LC-MS/MS in Abhängigkeit von der Inhibitorkonzentration gemessen und IC₅₀-Werte berechnet. Es wird der Einfluss auf die Metabolitenbildung bei einer definierten Substratkonzentration und 6 Konzentrationen der potentiellen Inhibitoren untersucht. Als Positiv-Kontrolle wird auf jeder Mikrotiterplatte ein bekannter Inhibitor der jeweils untersuchten CYP Isoform mitgeführt. Im Fall des CYP3A4 Assays wird zusätzlich die Untersuchung der Inhibition nach Vorinkubation der potentiellen Inhibitoren mit humanen Lebermikrosomen in Gegenwart von NADPH durchgeführt.

Experimentelle Details sind in der Tabelle C zusammengefasst:

**Tabelle C:**

| **CYP** | **Substrat** **(Konzentration)** | **Protein** **[mg/ml]** | **Inkubationszeit** **[min]** | **ISTD** | **Standard** **Inhibtor** |
|---|---|---|---|---|---|
| 1A2 | Phenacetin (30 µM) | 0.3 | 20 | D₃-acetaminophen | Fluvoxamine |
| 2C9 | Diclofenac (10 µM) | 0.05 | 15 | Tolbutamide | Sulfaphenazole |
| 2D6 | Dextromethorphan (10µm) | 0.5 | 20 | Quinidine | Fluoxetine |
| 3A4 | Midazolam (10 µM) | 0.2 | 15 | Oxazepam | Ketoconazole |
| mit Vorinkubation | | 0.2 | 15/15 | Oxazepam | Troleandomycin |

Die Inkubationen werden in Phosphat-Puffer (100 mM, pH 7.4) auf Mikrotiterplatten (96 well, 200 µl Volumen) durchgeführt. Inkubationszeit und Proteingehalt der Inkubationen sind abhängig vom verwendeten Substrat und in der Tabelle C zusammengefasst. Nach Zugabe von 100 µl Acetonitril (enthält den jeweiligen internen Standard) werden die gefällten Proteine mittels Zentrifugation über eine Filterplatte entfernt. Die Überstände werden vereinigt und die Proben mittels LC-MS/MS analysiert.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96%), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäßer Verbindung wird der Suspension zugefügt. Unter Rühren erfolgt die Zugabe des Wassers. Bis zum Abschluss der Quellung des Rhodigels wird ca. 6 Stunden gerührt.

## Patentansprüche

1. Verbindung der Formel (I) 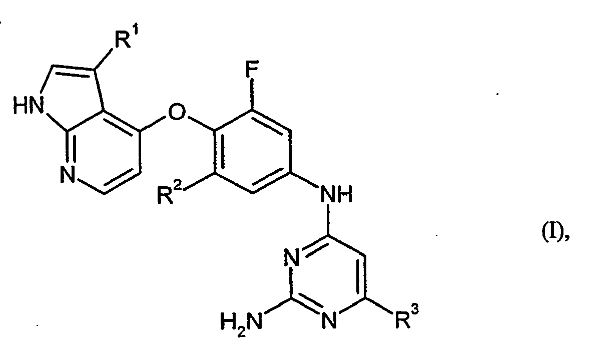 worin
R¹ Chlor, Brom, Cyano, Methyl, Ethyl, Hydroxyethyl, Methoxyethyl oder Cyclopropyl bedeutet,
R² Wasserstoff oder Fluor bedeutet,
R³ Wasserstoff, Chlor, Trifluormethyl oder Pentafluorethyl bedeutet,
oder eines ihrer Salze, ihrer Solvate oder der Solvate ihrer Salze.

2. Verbindung nach Anspruch 1,
worin
R¹ Chlor, Cyano, Methyl, Ethyl, Hydroxyethyl, Methoxyethyl oder Cyclopropyl bedeutet,
R² Wasserstoff oder Fluor bedeutet,
R³ Wasserstoff, Chlor oder Trifluormethyl bedeutet,
oder eines ihrer Salze, ihrer Solvate oder der Solvate ihrer Salze.

3. Verbindung nach Anspruch 1 oder 2,
worin
R¹ Cyano, Methyl oder Hydroxyethyl bedeutet,
R² Wasserstoff oder Fluor bedeutet,
R³ Chlor oder Trifluormethyl bedeutet,
oder eines ihrer Salze, ihrer Solvate oder der Solvate ihrer Salze.

4. Verfahren zur Herstellung einer Verbindung der Formel (I) wie in Anspruch 1 definiert, **dadurch gekennzeichnet, dass** man
eine Verbindung der Formel (II) 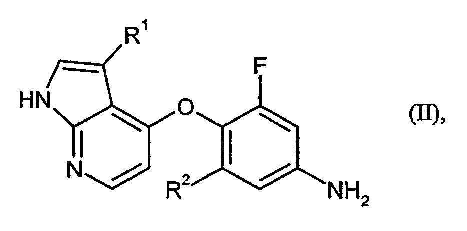 worin
R¹ und R² die in Anspruch 1 angegebene Bedeutung aufweisen,
mit einer Verbindung der Formel (III) 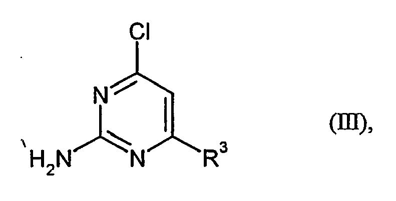 worin
R³ die in Anspruch 1 angegebene Bedeutung aufweist,
umsetzt.

5. Verbindung der Formel (I), wie in einem der Ansprüche 1 bis 3 definiert, zur Behandlung und/oder Prophylaxe von Erkrankungen.

6. Verwendung einer Verbindung der Formel (I), wie in einem der Ansprüche 1 bis 3 definiert, zur Herstellung von Arzneimitteln zur Behandlung und/oder Prophylaxe von kardiovaskulären Erkrankungen.

7. Verwendung einer Verbindung der Formel (I), wie in einem der Ansprüche 1 bis 3 definiert, zur Herstellung von Arzneimitteln zur Behandlung und/oder Prophylaxe von erektiler Dysfunktion.

8. Arzneimittel enthaltend eine Verbindung der Formel (I), wie in einem der Ansprüche 1 bis 3 definiert, in Kombination mit einem weiteren Wirkstoff.

9. Arzneimittel enthaltend eine Verbindung Formel (I), wie in einem der Ansprüche 1 bis 3 definiert, in Kombination mit einem inerten nichttoxischen, pharmazeutisch geeigneten Hilfsstoff.

10. Arzneimittel nach Anspruch 8 oder 9 zur Behandlung und/oder Prophylaxe von kardiovaskulären Erkrankungen.

11. Arzneimittel nach Anspruch 8 oder 9 zur Behandlung und/oder Prophylaxe von erektiler Dysfunktion.

## Claims

1. Compound of the formula (I) 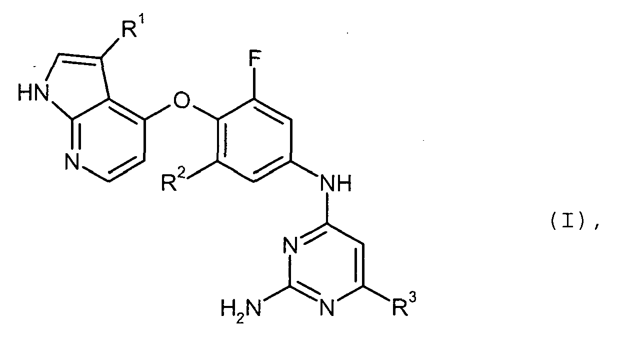 in which
R¹ represents chlorine, bromine, cyano, methyl, ethyl, hydroxyethyl, methoxyethyl or cyclopropyl,
R² represents hydrogen or fluorine,
R³ represents hydrogen, chlorine, trifluoromethyl or pentafluoroethyl,
or one of its salts, its solvates or the solvates of its salts.

2. Compound according to claim 1,
in which
R¹ represents chlorine, cyano, methyl, ethyl, hydroxyethyl, methoxyethyl or cyclopropyl,
R² represents hydrogen or fluorine,
R³ represents hydrogen, chlorine or trifluoromethyl,
or one of its salts, its solvates or the solvates of its salts.

3. Compound according to claim 1 or 2,
in which
R¹ represents cyano, methyl or hydroxyethyl,
R² represents hydrogen or fluorine,
R³ represents chlorine or trifluoromethyl,
or one of its salts, its solvates or the solvates of its salts.

4. Process for preparing a compound of the formula (I) as defined in claim 1, **characterized in that**
a compound of the formula (II) 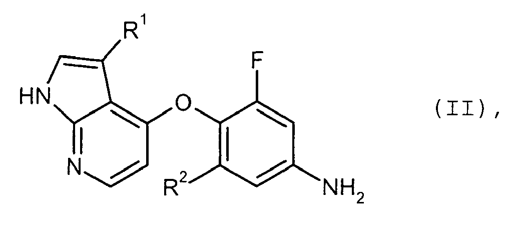 in which
R¹ and R² are as defined in claim 1
are reacted with a compound of the formula (III) 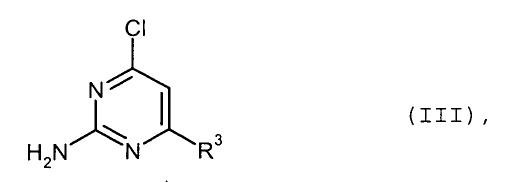 in which
R³ is as defined in claim 1.

5. Compound of the formula (I) as defined in any of claims 1 to 3 for the treatment and/or prophylaxis of disorders.

6. Use of a compound of the formula (I) as defined in any of claims 1 to 3 for preparing medicaments for the treatment and/or prophylaxis of cardiovascular disorders.

7. Use of a compound of the formula (I) as defined in any of claims 1 to 3 for preparing medicaments for the treatment and/or prophylaxis of erectile dysfunction.

8. Medicament, comprising a compound of the formula (I) as defined in any of claims 1 to 3 in combination with a further active compound.

9. Medicament comprising a compound of the formula (I) as defined in any of claims 1 to 3 in combination with an inert nontoxic pharmaceutically acceptable auxiliary.

10. Medicament according to claim 8 or 9 for the treatment and/or prophylaxis of cardiovascular disorders.

11. Medicament according to claim 8 or 9 for the treatment and/or prophylaxis of erectile dysfunction.

## Revendications

1. Composé de formule (I) 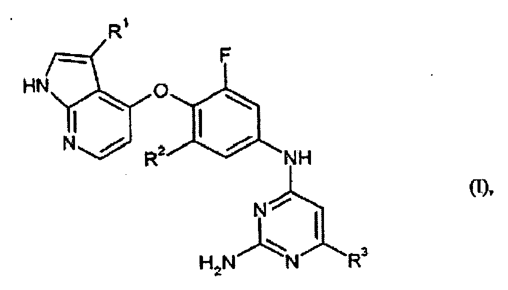 dans laquelle
R¹ représente le chlore, le brome, un groupe cyano, un reste méthyle, éthyle, hydroxyéthyle, méthoxyéthyle ou cyclopropyle,
R² représente l'hydrogène ou le fluor,
R³ représente l'hydrogène, le chlore, un reste trifluorométhyle ou pentafluoréthyle,
ou l'un de ses sels, de ses produits de solvatation ou des produits de solvatation de ses sels.

2. Composé suivant la revendication 1,
dans lequel,
R¹ représente le chlore, un groupe cyano, un reste méthyle, éthyle, hydroxyéthyle, méthoxyéthyle ou cyclopropyle,
R² représente l'hydrogène ou le fluor,
R³ représente l'hydrogène, le chlore ou un reste trifluorométhyle,
ou l'un de ses sels, de ses produits de solvatation ou des produits de solvatation de ses sels.

3. Composé suivant la revendication 1 ou 2,
dans lequel
R¹ représente un groupe cyano, un reste méthyle ou hydroxyéthyle,
R² représente l'hydrogène ou le fluor,
R³ représente le chlore ou un reste trifluorométhyle,
ou l'un de ses sels, de ses produits de solvatation ou des produits de solvatation de ses sels.

4. Procédé de production d'un composé de formule (I) telle que définie dans la revendication 1, **caractérisé en ce qu'**on fait réagir
un composé de formule (II) 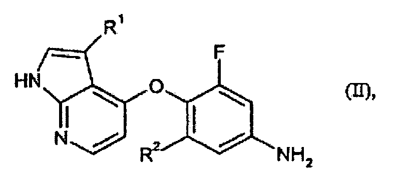 dans laquelle
R¹ et R² ont la définition indiquée dans la revendication 1, avec un composé de formule (III) 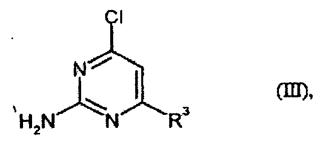 dans laquelle
R³ a la définition indiquée dans la revendication 1.

5. Composé de formule (I) telle que définie dans l'une des revendications 1 à 3, destiné au traitement et/ou à la prophylaxie de maladies.

6. Utilisation d'un composé de formule (I) telle que définie dans l'une des revendications 1 à 3 pour la préparation de médicaments destinés au traitement et/ou à la prophylaxie de maladies cardiovasculaires.

7. Utilisation d'un composé de formule (I) telle que définie dans l'une des revendications 1 à 3 pour la préparation de médicaments destinés au traitement et/ou à la prophylaxie d'un dysfonctionnement de l'érection.

8. Médicament contenant un composé de formule (I) telle que définie dans l'une des revendications 1 à 3 en association avec une autre substance active.

9. Médicament contenant un composé de formule (I) telle que définie dans l'une des revendications 1 à 3 en association avec une substance auxiliaire inerte non toxique pharmaceutiquement acceptable.

10. Médicament suivant la revendication 8 ou 9, destiné au traitement et/ou à la prophylaxie de maladies cardio-vasculaires.

11. Médicament suivant la revendication 8 ou 9, destiné au traitement et/ou à la prophylaxie d'un dysfonctionnement de l'érection.
